# EUROPEAN PATENT APPLICATION

(11) **EP 4 837 918 A1**
(43) Date of publication of application: **11.11.2026**
(21) Application number: 25174871.1
(22) Date of filing: 07.05.2025
(51) Int. Cl.: C07K 16/18, G01N 21/552, G01N 33/543

(54) **SURFACE FUNCTIONALIZED OPTICAL ELEMENT AND METHODS OF USE THEREOF**

(71) Applicant: betaSENSE GmbH, 44799 Bochum (DE)
(72) Inventor: SIMON, Jonas, 44791 Bochum (DE); LANGENHOFF, Lennart Carl, 44789 Bochum (DE); HUBERT, Diana, 44869 Bochum (DE); GEWERT, Klaus, 48147 Bochum (DE)
(74) Representative: Maiwald GmbH

(57) **Abstract**

The present invention relates to a surface-functionalized optical element comprising an IR-transparent optical element, wherein at least a part of the surface of the IR-transparent optical element is surface-functionalized, wherein said surface functionalization comprises a surface attachment moiety covalently attached to the surface of the IR-transparent optical element, and wherein at least part of the surface attachment moieties is covalently attached to a spacer moiety comprising a poly(alkylene oxide), wherein at least part of the spacer moieties is covalently attached to a charge optimization moiety, wherein said charge optimization moiety comprises a functional group that is charged under conditions of use of the optical element and optionally a poly(alkylene oxide), wherein at least part of the charge optimization moiety is covalently attached to a linker moiety comprising a poly(alkylene oxide), wherein at least part of the linker moieties is covalently attached to a molecular probe. The invention further relates to a single chain variable fragment (scFv) that can be used as a molecular probe for the capture of alpha-synuclein as well as sensor elements and sensors comprising said surface-functionalized optical element and/or scFv, and methods and uses thereof.

## Description

### FIELD OF THE INVENTION

The present invention relates to the field of molecular diagnostics and more specifically surface-functionalized optical elements such as surface-functionalized ATR-IR waveguides and optical biosensors which comprise such surface-functionalized optical elements. It further relates to methods for detecting a target analyte or determination of the protein conformational states, in particular the proportion of alpha-synuclein fibrils/aggregates in a sample, as well as the related uses.

### BACKGROUND

Neurodegenerative diseases that are associated with intracellular deposits of alpha-synuclein are commonly referred to as synucleinopathies. While the most well-known of these synucleinopathies is Parkinson's disease (PD), there are a variety of other diseases including, but not limited to, Lewy Body dementia (LBD; dementia with Lewy bodies (DLB) ("pure" Lewy body dementia), Parkinson's disease dementia (PDD)), diffuse Lewy body disease (DLBD), sporadic Alzheimer's disease, familial Alzheimer's disease with APP mutations, familial Alzheimer's disease with PS-1, PS-2 or other mutations, familial British dementia, Lewy body variant of Alzheimer's disease, multiple system atrophy (MSA) and Down syndrome.

Alpha-synuclein is a 140 amino acid long, cytosolic protein abundantly and predominantly expressed in the CNS and localized in pre-synaptic terminals. Alpha-synuclein is a natively unfolded protein but adopts secondary structure of mostly helical nature upon association with lipid vesicles or membranes and can also misfold and aggregate into larger oligomeric and fibrillar forms which are linked to the pathogenesis of synucleinopathies, such as PD. The C-terminal part of alpha-synuclein is the part that undergoes essentially all of the known post-translational modifications. There is evidence that some of these modifications, in particular the phosphorylation, glycosylation, nitration and C-terminal truncation, may affect fibril formation and aggregation, as all of these have been detected in Lewy bodies and/or alpha-synuclein aggregates.

Parkinson's disease (PD) is the most common neurodegenerative motor disorder. PD is mainly an idiopathic disease, although in at least 5% of the PD patients the pathology is linked to mutations in one or several specific genes. The pathogenesis of PD remains elusive, however, growing evidence suggests a role for the pathogenic folding of the alpha-synuclein protein that leads to the formation of amyloid-like fibrils. Indeed, the hallmarks of PD are the presence of intracellular alpha-synuclein aggregate structures called Lewy Bodies in the nigral neurons, as well as the death of dopaminergic neurons in the substantia nigra and elsewhere.

Studies have implicated small soluble oligomeric and protofibrillar forms of alpha-synuclein as the most neurotoxic species. Evidence from cellular and animal models does suggest that pathological and/or aggregated alpha-synuclein can spread from one neuron to another. Once inside the new cell alpha-synuclein aggregates act as seeds, recruiting endogenous alpha-synuclein and advancing protein aggregation. The transsynaptic spreading of pathological and/or aggregated alpha-synuclein could explain the progressive advancing of Lewy pathology through defined anatomical connected brain areas in PD.

To date, the diagnosis of Parkinson's disease is still largely clinical and depends on the presence of a specific set of symptoms and signs (the initial core feature being bradykinesia, rigidity, rest tremor and postural instability), a slowly progressive course, and a response to drug treatment. The final confirmation of the diagnosis is made by post-mortem neuropathological analysis.

Recent evidence suggests that alpha-synuclein misfolding already occurs in very early stages of the disease, many years before the disease clinically manifests. As the detection of such early stages may provide new treatment options and also be a means for risk stratification for developing a synucleinopathy in the future, there is an ongoing need for novel methods that allow sensitive determination of misfolded alpha-synuclein at low levels, long before any disease symptoms occur.

Although biomarkers for PD, including alpha-synuclein, have been investigated in different body fluids (cerebrospinal fluid (CSF), plasma, saliva), so far none of these biomarkers alone or in combination has been established for use as a determinant diagnostic test. This may be at least partially related to the fact that techniques such as enzyme-linked immune-sorbent assays (ELISA), surface plasmon resonance spectroscopy (SPR), surface fluorescence intensity distribution analysis (sFIDA) or mass spectroscopy, usually do not provide direct information about the secondary structure of the analytes, and, if they do, can typically only detect one of the multiple forms of the analyte. Other (spectroscopic) techniques are hampered by the need for highly purified protein samples that may be difficult to obtain with reasonable efforts, in particular if sample volumes and analyte concentrations are low.

Infrared attenuated total reflection spectroscopy has been reported for analysis of amyloid beta and related proteins in biological fluids (WO 2015/121339 A1; Nabers et al., Analytical Chemistry, 88: 2755-2762 (2016)), using surface-functionalized optical elements to capture and analyze the target analyte. Surface-functionalized optical elements find widespread use in biosensing applications or biosensors. In said applications, a molecular probe - e.g. an antibody- is immobilized on the surface of the optical element.. The chemical/(biochemical) interaction between the molecular probe and the target analyte produces a measurable signal that is specific for the respective target analyte. The measured signal results from a physical-chemical change, which can be measured by optical spectroscopy via the optical element which serves as a so-called transduction element.

In known surface-functionalized optical elements, it remains challenging to prevent nonspecific binding and reduce background noise. For example, the inherent surface charge of the surface of the optical elements may attract oppositely charged molecules from the sample solution, which leads to nonspecific (protein) adsorption to the surface of the optical element and thus create signal noise in the measurement. Positively charged proteins e.g. the lysozyme and negatively charged molecules e.g. all kinds of nucleic acids are examples of molecules prone for nonspecific protein adsorption to charged surfaces depending on the net charge of the surface. Existing surface modification techniques using functional groups and/or molecular probes are in many cases incapable of sufficiently reducing such nonspecific, charge-mediated adsorption. There is thus need in the art for alternative surface-functionalization that allows further reduction of nonspecific adsorption and thus allows a better signal to noise ratio.

Furthermore, this approach, while being highly suitable for analyzing the secondary structure profile of peptides potentially transitioning between several conformational states, is critically dependent on the availability of suitable molecular probes, such as capture antibodies, which recognize and specifically bind the intended target, such as alpha-synuclein, substantially independent of its conformational state. It further depends on the provision of surface-functionalized optical elements suitable for infrared attenuated total reflection spectroscopy in which said molecular probes, typically antibodies or antibody-like molecules, are covalently attached to the optical element while also reducing nonspecific protein adsorption to minimize signal noise.

### SUMMARY OF INVENTION

The present invention meets all of the above needs. The inventors surprisingly found that by adjusting the net charge of the surface of the optical element using a specific type of surface functionalization as defined herein, the surface-functionalized optical element is less prone to nonspecific protein adsorption. As a result the recorded signal comprises less noise than that of a surface-functionalized optical element which uses a different linker for binding the molecular probe to the surface.

In a first aspect, the invention is therefore directed to a surface-functionalized optical element, which comprises an IR-transparent optical element,
wherein at least a part of the surface of the IR-transparent optical element is surface-functionalized, wherein said surface functionalization comprises
a surface attachment moiety covalently attached to the surface of the IR-transparent optical element, wherein at least part of the surface attachment moieties is covalently attached to a spacer moiety comprising a poly(alkylene oxide),
wherein at least part of the spacer moieties is covalently attached to a charge optimization moiety, wherein said charge optimization moiety comprises a functional group that is charged under conditions of use of the optical element and optionally a poly(alkylene oxide),
wherein at least part of the charge optimization moiety is covalently attached to a linker moiety comprising a poly(alkylene oxide),
wherein at least part of the linker moieties is covalently attached to a molecular probe.

The surface-functionalized optical element thus comprises (1) surface attachment moieties, (2) spacer moieties, (3) charge optimization moieties, (4) linker moieties and (5) molecular probes, as defined herein.

In various embodiments of the surface-functionalized optical element said charge optimization moiety comprises a functional group that is at least charged, preferably positively charged, if said charge optimization moiety is not covalently attached to a linker moiety.

In various embodiments, at least part but not all of the charge optimization moieties are covalently attached to a linker moiety. The number of the charge optimization moieties not covalently attached to a linker moiety may be adjusted such that the net charge of the surface of the surface-functionalized optical element is about zero.

In various embodiments, said surface functionalization comprises a charge optimization construct having the structure

//-(S)-(SP)-(CO)

wherein
// represents attachment to the surface of the IR-transparent optical element,
(S) represents the surface attachment moiety,
(SP) represents the spacer moiety,
(CO) represents the charge optimization moiety,
wherein said charge optimization moiety comprises a functional group that is charged under conditions of use of the optical element.

In various embodiments, said surface functionalization comprises a molecular probe linker construct having the structure

//-(S)-(SP)-(CO)-(L)-\\

wherein
// represents attachment to the surface of the IR-transparent optical element,
(S) represents the surface attachment moiety,
(SP) represents the spacer moiety,
(CO) represents the charge optimization moiety,
(L) represent the linker moiety, and
\\ represents attachment to another moiety of the molecular probe linker construct or to the molecular probe.

In various embodiments, the surface functionalization comprises both, a molecular probe linker construct having the structure

//-(S)-(SP)-(CO)-(L)-\\

and a charge optimization construct having the structure

//-(S)-(SP)-(CO).

In various embodiments, the surface attachment moiety (S) has the structure

//-Y¹-L¹-X¹-$$¹

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is an optional linking unit,
X¹ is a group selected from -C(O)NH-, -NHC(O)-, -NHC(O)HN-, and -NHC(O)O-,
$$¹ represents attachment to the spacer moiety.

In various embodiments, the spacer moiety (SP) has the structure

$$²-L²-X²-L³-H¹-&&²

wherein
$$² represents attachment to the surface attachment moiety (S),
L² and L³ are each independently an optional linking unit,
X² is -(AO)n¹-, wherein AO is an alkylene oxide, preferably ethylene oxide (EO) or propylene oxide (PO), more preferably ethylene oxide, and n¹ is an integer from 2 to 25, preferably from 2 to 10, more preferably from 2 to 8, most preferably from 3 to 5,
H¹ is an optionally substituted N-heterocyclic group having a structure according to any one of formulae (HET-I) to (HET-VI): 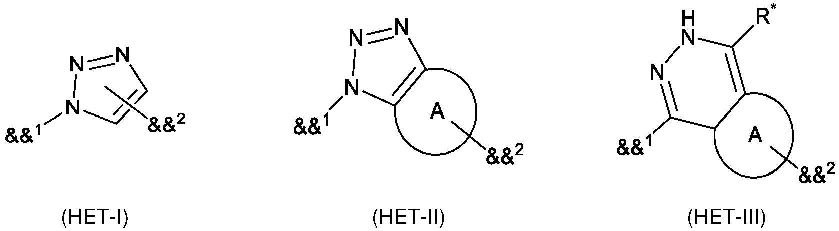 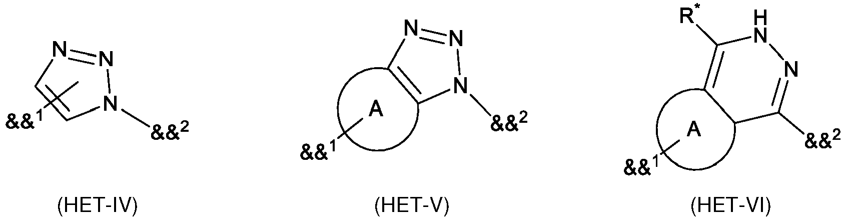 wherein
   &&¹ represents attachment to L³ or, if L³ is absent, to X²,
   &&² represents the attachment to the charge optimization moiety (CO),
   R* is H, an optionally substituted alkyl group or an optionally substituted aryl group, and preferably is a C₁-C₆ alkyl group,
   ring A is an optionally substituted 8- to 9-membered mono-, bi- or multicyclic carbocycle or an optionally substituted 8- to 9-membered mono-, bi- or multicyclic heterocycle, and is preferably an optionally substituted dibenzo-fused 8-membered N-heterocycle or an optionally substituted bicyclo[6.1.0]non-4-ene ring, more preferably an optionally substituted bicyclo[6.1.0]non-4-ene ring.

In various embodiments, the charge optimization moiety (CO) has the structure

&&³-L⁴-X³-L⁵-X⁴-L⁶-C¹

wherein
&&³ represents attachment to the spacer moiety (SP),
L⁴, L⁵ and L⁶ are each independently an optional linking unit,
X³ is a group selected from -C(O)NH-, -NHC(O)-, -NHC(O)HN-, -NHC(O)O-, and -OC(O)NH-, X⁴ is absent or, preferably, is -(AO)n²-, wherein AO is an alkylene oxide, preferably ethylene oxide (EO) or propylene oxide (PO), more preferably ethylene oxide, and n² is an integer from 1 to 25, preferably from 2 to 10, more preferably from 2 to 5, most preferably 2 to 4,
C' is (1) a charged functional group, preferably an amine group, more preferably a primary amine group, or (2) has the structure

   X⁶-##¹

   wherein X⁶ is covalently attached to a L⁶ and is a group selected from -C(O)NH-, -NHC(O)-, -NHC(O)HN-, -NHC(O)O-, and -OC(O)NH-, and
   ##¹ represents attachment to the linker moiety (L). In various embodiments, the charge optimization moiety (CO) has the structure -(CH₂)₁₋₂-OC(O)-NH-(CH₂CH₂O)₂₋₄-(CH₂)₂₋₄-NH₃⁺ or -(CH₂)₁₋₂-OC(O)-NH-(CH₂CH₂O)₂₋₄-(CH₂)₂₋₄-NHC(O)-##¹.

In various embodiments, the linker moiety (L) has the structure

##²-L⁷-X⁷-L¹⁰-H²-L¹¹-**

or

(##²-L⁷-X⁷-L⁸)₂X⁸-L⁹-X⁹-L¹⁰- H²-L¹¹-**

wherein
each ##² represents attachment to the charge optimization moiety (CO),
L⁷, L⁸, L⁹, L¹⁰ and L¹¹ are each independently an optional linking unit,
X⁷ is -(AO)n³-, wherein AO is an alkylene oxide, preferably ethylene oxide (EO) or propylene oxide (PO), more preferably ethylene oxide, and n³ is an integer from 1 to 25, preferably from 2 to 10, more preferably from 3 to 9,
X⁸ is NC(O)-, NC(O)NH- or NC(O)O-,
X⁹ is -(AO)n⁴-, wherein AO is an alkylene oxide, preferably ethylene oxide (EO) or propylene oxide (PO), more preferably ethylene oxide, and n⁴ is an integer from 1 to 25, preferably from 2 to 10, more preferably from 2 to 5, most preferably 2 to 4,
wherein the sum of all n³ and n⁴ is preferably 4 to 25, more preferably 5 to 12,
H² is an optionally substituted N-heterocyclic group having a structure according to any one of formulae (HET-I) to (HET-VI): 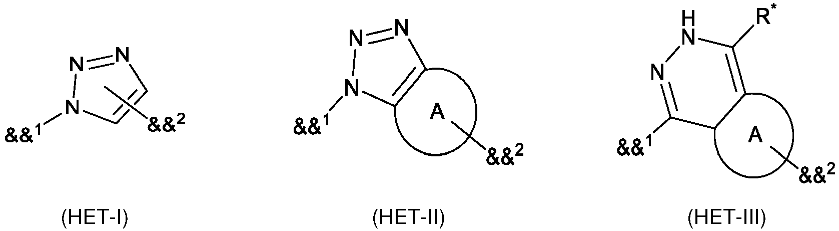 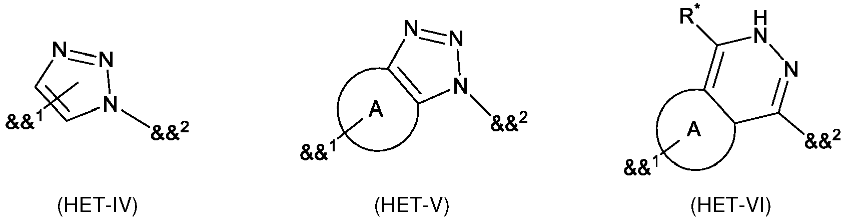 wherein
   &&¹ represents attachment to L¹⁰ or, if L¹⁰ is absent, to X⁹,
   &&² represents the attachment to L¹¹ or, if L¹¹ is absent, corresponds to **,
   R* is H, an optionally substituted alkyl group or an optionally substituted aryl group, and preferably is a C₁-C₆ alkyl group,
   ring A is an optionally substituted 8- to 9-membered mono-, bi- or multicyclic carbocycle or an optionally substituted 8- to 9-membered mono-, bi- or multicyclic heterocycle, and is preferably an optionally substituted dibenzo-fused 8-membered N-heterocycle or an optionally substituted bicyclo[6.1.0]non-4-ene ring, more preferably an optionally substituted dibenzo-fused 8-membered N-heterocycle, and
   ** represents the attachment to another moiety of the molecular probe linker construct or to the molecular probe. The sum of all n¹, n², n³, and n⁴ (i.e. n¹ + n² + n³ + n⁴) of the molecular probe linker construct may be in the range of 10 to 25, preferably 12 to 18.

In various embodiments, said surface functionalization further comprises a surface functionalization moiety (SFM) having the structure

//-Y¹-L¹-X¹-L^{1a}-X^{1a}-M¹

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of another matrix silane being attached to the surface of the IR-transparent optical element,
L¹ and L^{1a} are each independently an optional linking unit,
X¹ is a group selected from -C(O)NH-, -NHC(O)-, -NHC(O)HN-, and -NHC(O)O-,
X^{1a} is -(AO)ₙ₁ₐ-, wherein AO is an alkylene oxide, preferably ethylene oxide (EO) or propylene oxide (PO), more preferably ethylene oxide, and n^{1a} is an integer from 2 to 25, preferably from 2 to 10, more preferably from 2 to 8, most preferably from 2 to 5 or 2 to 4, and
M¹ is a C₁-C₆ alkyl group, preferably methyl.

In various embodiments, the IR-transparent optical element is an attenuated total reflection infrared (ATR-IR) waveguide.

In various embodiments, at least a part of the IR-transparent optical element has an oxide surface layer, preferably a silicon oxide surface layer, and wherein at least a part of the oxide surface layer surface functionalized.

In various embodiments, the molecular probe is an antigen, an antibody, a fragment of an antibody, an antibody fusion protein or fusion proteins with antibody fragments, an antibody conjugate or conjugates with antibody fragments, a protein complex which optionally contains an antibody fragment or a fusion protein thereof, an anticalin, a nanobody, a small organic molecule, a drug, a nucleic acid, an aptamer, a lipid, a carbohydrate, or a peptide, and/or the molecular probe is capable of forming a complex with a protein which is associated with a protein misfolding disease.

In another aspect, the invention is directed to an optical biosensor or an infrared sensor element comprising the surface-functionalized optical element of the invention or the single chain variable fragment as disclosed herein below.

In still another aspect, the invention is directed to a process for preparing a surface-functionalized optical element according to the invention, wherein the process comprises the steps of:
(a) providing an IR-transparent optical element,
(b) reacting at least a part of the surface of the IR-transparent optical element with a first compound to form an optical element surface-functionalized with a surface attachment moiety, wherein said surface attachment moiety comprises a reactive group,
(c) reacting the optical element surface-functionalized with a surface attachment moiety comprising a reactive group with a second compound to form an optical element surface-functionalized with a surface attachment moiety and a spacer moiety covalently attached to the surface attachment moiety, wherein the spacer moiety comprises a reactive group,
(d) reacting the optical element surface-functionalized with a surface attachment moiety and a spacer moiety comprising a reactive group with a third compound to form an optical element surface-functionalized with a surface attachment moiety, a spacer moiety and a charge optimization moiety covalently attached to the spacer moiety, wherein the charge optimization moiety comprises a charged functional group,
(e) reacting the optical element surface-functionalized with a surface attachment moiety, a spacer moiety and a charge optimization moiety comprising a charged functional group with a fourth compound to form an optical element surface-functionalized with a surface attachment moiety, a spacer moiety, a charge optimization moiety and a linker moiety covalently attached to the charge optimization moiety, wherein the linker moiety comprises a reactive group,
(f) reacting the optical element surface-functionalized with a surface attachment moiety, a spacer moiety, a charge optimization moiety and a linker moiety comprising a reactive group with a molecular probe to form an optical element surface-functionalized with a surface attachment moiety, a spacer moiety, a charge optimization moiety, a linker moiety and a molecular probe.

In a further aspect, the invention is directed to a method for detecting a biomarker, comprising the steps of
(i) bringing into contact a surface-functionalized optical element or infrared sensor element according to the invention with a sample suspected to comprise a target analyte, and optionally wherein the sample is a human body fluid;
(ii) detecting the biomarker based on an interaction between the molecular probe and the target analyte, and optionally wherein the interaction between the molecular probe and the target analyte is detected by infrared spectroscopy, and optionally wherein the biomarker is based on an amide I band position of the target analyte.

In a still further aspect, the invention is directed to the use of a surface-functionalized optical element or infrared sensor element according to the invention or of an optical biosensor according to the invention for one or more of:
a companion diagnostic test,
diagnosing a protein misfolding disease, diabetes or a tumor in a patient,
monitoring of therapy of patients having a protein misfolding disease, diabetes or a tumor, and for screening of drugs for the treatment of a protein misfolding disease, diabetes or a tumor.

Another aspect of the present invention is based on the inventors' design of a specific antibody molecule that is particularly well suited for the detection of alpha-synuclein in a sample. Said aspect therefore relates to a method for determining the proportion of alpha-synuclein in fibril and/or aggregated form relative to the total amount of alpha-synuclein in a sample obtained from a human subject, comprising:
(a) obtaining an IR spectrum of the sample in a cell comprising an infrared sensor element linked to an antibody capable of binding to alpha-synuclein independent of whether it is in its monomeric or its fibril/aggregated form, wherein the antibody is a single chain antibody (scFv) and comprises three variable heavy chain complementarity determining regions VH-CDR1, VH-CDR2 and VH-CDR3 and three variable light chain complementarity determining regions VL-CDR1, VL-CDR2 and VL-CDR3, wherein VH-CDR1 comprises the amino acid sequence of SEQ ID NO:2, VH-CDR2 comprises the amino acid sequence of SEQ ID NO:3, VH-CDR3 comprises the amino acid sequence of SEQ ID NO:4, VL-CDR1 comprises the amino acid sequence of SEQ ID NO:5, VL-CDR2 comprises the amino acid sequence of SEQ ID NO:6, and VL-CDR3 comprises the amino acid sequence of SEQ ID NO:7;
(b) determining a value in the obtained infrared spectrum indicative for the proportion of alpha-synuclein in fibril and/or aggregated form relative to the total amount of alpha-synuclein in the sample.

In various embodiments, said scFv binds to an epitope within amino acid residues 126-140 of human alpha-synuclein using the numbering according to SEQ ID NO:1.

In various embodiments, the scFv antibody comprises a heavy chain variable domain (V_{H}) having an amino acid sequence that has at least 90%, preferably 100%, sequence identity to the amino acid sequence set forth in SEQ ID NO:8 over its entire length and a light chain variable domain (V_{L}) having an amino acid sequences that has at least 90%, preferably 100%, sequence identity to the amino acid sequence set forth in SEQ ID NO:9 over its entire length, wherein the heavy chain variable region and the light chain variable region are covalently linked by a peptide linker of 2 to 50 amino acids in length.

In various embodiments, the scFv comprises the structure, from N to C-terminus, V_{H}-linker-V_{L}, wherein the linker is an amino acid sequence covalently linking the V_{H} and V_{L} domain, as defined herein, which is preferably 2 to 50 amino acids in length, more preferably 5 to 30, even more preferably 10 to 25 amino acids in length, which may, in various embodiments, be rich in G and S residues, in particular the GGS motif, for example 4 to 10 copies of the GGS motif, for example having the amino acid sequence as set forth in SEQ ID NO:12.

In various embodiments, the scFv antibody comprises, from N- to C-terminus, the amino acid sequence: SEQ ID NO:8-SEQ ID NO:12-SEQ ID NO:9, covalently linked to each other by peptide bonds.

In various embodiments, the ScFv antibody comprises or consists of the amino acid sequence set forth in SEQ ID NO:10.

In various embodiments, the method further comprises step (a1) of contacting the sample prior to step (a) with a variant of the antibody of step (a), wherein said variant antibody is mutated to completely abolish its binding to alpha-synuclein but wherein said antibody has at least 90%, preferably at least 95 % sequence identity relative to the antibody used in step (a) over its entire length. The variant antibody may comprise any one or all, preferably all, of the mutations A3F/D in SEQ ID NO:1, R1D in SEQ ID NO:2 and G3D in SEQ ID NO:3 but is otherwise identical to the antibody used in step (a).

In various embodiments, the value indicative for the proportion of alpha-synuclein in fibril and/or aggregated form determined in step (b) is
(b1) an amide I band maximum;
(b2) an amide I band center of mass;
(b3) a ratio of signal at 1650 cm⁻¹ to signal at 1630 cm⁻¹;
(b4) a ratio of signal at 1656 cm⁻¹ to signal at 1623.5 cm⁻¹; or
(b5) a ratio of signal at 1651.5 cm⁻¹ to signal at 1623.5 cm⁻¹.

In various embodiments, step (b) comprises comparing the obtained IR spectrum with a reference IR spectrum of alpha-synuclein, wherein optionally the reference IR spectrum is obtained from a sample with a known proportion of alpha-synuclein in fibril and/or aggregated form.

In various embodiments, the method further comprises:
(c) based on the value indicative for the proportion of alpha-synuclein in fibril and/or aggregated form relative to the total amount of alpha-synuclein in the sample, determining whether the human subject is afflicted by or has a risk of developing a disease or disorder characterized by alpha-synuclein aggregation. In such a method, (c1) an amide I band maximum below 1639 cm⁻¹, optionally below 1638 cm⁻¹, (c2) an amide I band center of mass of below 1641 cm⁻¹, optionally below 1640 cm⁻¹, (c3) a ratio of the signal at 1650 cm⁻¹ to the signal at 1630 cm⁻¹ below 0.99, optionally below 0.98, (c4) a ratio of the signal at 1656 cm⁻¹ to the signal at 1623.5 cm⁻¹ below 1.15, optionally below 1.10, and/or (c5) a ratio of the signal at 1651.5 cm⁻¹ to the signal at 1623.5 cm⁻¹ below 1.20, optionally below 1.15
may be indicative for a disease or disorder characterized by alpha-synuclein aggregation or for an increased risk of developing a disease or disorder characterized by alpha-synuclein aggregation.

In various embodiments, (c3) a ratio of the signal at 1656 cm⁻¹ to the signal at 1623.5 cm⁻¹ below 1.065 is indicative for a disease or disorder characterized by alpha-synuclein aggregation or for an increased risk of developing a disease or disorder characterized by alpha-synuclein aggregation. In various embodiments, (c3) a ratio of the signal at 1656 cm⁻¹ to the signal at 1623.5 cm⁻¹ of greater than 1.14 is indicative for not being afflicted by a disease or disorder characterized by alpha-synuclein aggregation or for low risk of developing a disease or disorder characterized by alpha-synuclein aggregation. In various embodiments, (c3) a ratio of the signal at 1656 cm⁻¹ to the signal at 1623.5 cm⁻¹ of equal to or lower than 1.14 and equal to and higher than 1.065 is indicative for not being clearly afflicted by a disease or disorder characterized by alpha-synuclein aggregation but for having an elevated risk of developing a disease or disorder characterized by alpha-synuclein aggregation.

In various embodiments of the methods, (1) the sample is a sample obtained from a human subject suspected of being afflicted by a disease or disorder characterized by alpha-synuclein aggregation or at risk of developing such a disease or disorder, wherein the disease or disorder is optionally selected from Lewy body disease and Parkinson's disease; and/or
(2) the sample is a cerebrospinal fluid sample, a blood sample, or a tissue homogenate including brain and skin.

In a still further aspect, the invention is directed to an infrared sensor element for determining the proportion of alpha-synuclein in fibril and/or aggregated form relative to the total amount of alpha-synuclein in a sample obtained from a human subject, comprising an antibody capable of binding to alpha-synuclein independent of whether it is in its monomeric or its fibril/aggregated form, wherein said antibody is the single chain variable fragment antibody of the present invention.

In a still further aspect, the invention is directed to a device for determining the proportion of alpha-synuclein in fibril and/or aggregated form relative to the total amount of alpha-synuclein of alpha-synuclein in a sample obtained from a human subject, comprising:
(a) an infrared source,
(b) an infrared sensor element according to the present invention, and
(c) an infrared detector.

The invention is further directed, in another aspect, to the use of a scFv antibody comprising three variable heavy chain complementarity determining regions VH-CDR1, VH-CDR2 and VH-CDR3 and three variable light chain complementarity determining regions VL-CDR1, VL-CDR2 and VL-CDR3, wherein VH-CDR1 comprises the amino acid sequence of SEQ ID NO:2, VH-CDR2 comprises the amino acid sequence of SEQ ID NO:3, VH-CDR3 comprises the amino acid sequence of SEQ ID NO:4, VL-CDR1 comprises the amino acid sequence of SEQ ID NO:5, VL-CDR2 comprises the amino acid sequence of SEQ ID NO:6, and VL-CDR3 comprises the amino acid sequence of SEQ ID NO:7, for determining the proportion of alpha-synuclein in fibril and/or aggregated form relative to the total amount of alpha-synuclein in a sample obtained from a human subject.

In a still further aspect, the invention is directed to the use of the optical biosensor, infrared sensor element or the device of the invention for
(a) determining the proportion of alpha-synuclein in fibril and/or aggregated form relative to the total amount of alpha-synuclein in a sample obtained from a human subject; or
(b) for detecting a disease or disorder characterized by alpha-synuclein aggregation or for determining the risk of developing a disease or disorder characterized by alpha-synuclein aggregation by determining the proportion of alpha-synuclein in fibril and/or aggregated form relative to the total amount of alpha-synuclein in a sample obtained from a human subject.

In a still further aspect, the invention is directed to a single chain variable fragment (scFv) comprising, from N- to C-terminus, the amino acid sequence: SEQ ID NO:8-SEQ ID NO:12-SEQ ID NO:9, covalently linked to each other by peptide bonds, or comprising or consisting of the amino acid sequence of SEQ ID NO:10.

### BRIEF DESCRIPTION OF THE DRAWINGS

Figure 1 schematically shows the structure of a conventional casein-based blocking surface and the peptide free and charge optimized amine surface according to the invention.
Figure 2 shows ATR-FTIR spectra of nonspecific Serodos^{®} binding without antibody (A) and with immobilized antibody dead mutant (B) on casein-based blocking surface (grey) and amine surface (black).
Figure 3 shows ATR-FTIR spectra of different serum control proteins on the inventive amine surface.
Figures 4 shows the surface attraction of different proteins to the casein-based blocking surface (black), a BSA-free peptide blocking surface (grey) and the protein free Amine surface (white).
Figure 5 shows protein binding (black) and corresponding amide-I band maxima (grey) on different surface combinations (A). Protein binding (black) and corresponding alpha-Synuclein reduction (grey) on different surface combinations (B).
Figure 6 shows fluorescence polarisation measurements of the different anti α-Synuclein scFv fragments and non-binding (dead) mutants.
Figure 7 shows binding of target analyte in different amounts to casein-based blocking surface (black) and protein-free amine surface (white).

### DETAILED DESCRIPTION

The terms used herein have, unless explicitly stated otherwise, the meaning as commonly understood in the art.

Where the term "comprising" is used in the present description and claims, it does not exclude other non-specified elements of major or minor functional importance. For the purposes of the present invention, the term "essentially consisting of" and "consisting of" are considered to be specific embodiments of the term "comprising of". If hereinafter a group is defined to comprise at least a certain number of features or embodiments, this is also to be understood to disclose a group, which optionally essentially consists only of these features or embodiments or consists only of these features or embodiments.

Whenever the terms "including" or "having" are used, these terms are meant to be equivalent to "comprising" as defined above. Where an indefinite or definite article is used when referring to a singular noun, e.g. "a", "an" or "the", this includes a plural of that noun unless something else is specifically stated. The term "obtained" does not mean to indicate that, e.g., an embodiment must be obtained by, e.g., the sequence of steps following the term "obtained" even though such a limited understanding is always included by the terms "obtained" as a preferred embodiment.

### Surface-functionalized optical element

In one aspect of the invention, a surface-functionalized optical element is provided. The surface-functionalized optical element comprises an IR-transparent optical element, wherein at least a part of the surface of the IR-transparent optical element is surface-functionalized, wherein said surface functionalization comprises a charge optimization moiety, wherein said charge optimization moiety comprises a functional group that is charged under conditions of use of the optical element and optionally a poly(alkylene oxide).

Said surface functionalization typically comprises a surface attachment moiety covalently attached to the surface of the IR-transparent optical element, and
wherein at least part of the surface attachment moieties is covalently attached to a spacer moiety comprising a poly(alkylene oxide),
wherein at least part of the spacer moieties is covalently attached to a charge optimization moiety, wherein said charge optimization moiety comprises a functional group that is charged under conditions of use of the optical element and optionally a poly(alkylene oxide),
wherein at least part of the charge optimization moiety is covalently attached to a linker moiety comprising a poly(alkylene oxide),
wherein at least part of the linker moieties is covalently attached to a molecular probe.

This means that the surface functionalization generally comprises all of (1) surface attachment moieties, (2) spacer moieties, (3) charge optimization moieties, (4) linker moieties and (5) molecular probes, as defined herein. However, not all surface attachment moieties need to be covalently attached to a spacer moiety. In fact, in various embodiments, at least part of the surface attachment moieties are not attached to a spacer moiety. This means that the surface functionalization comprises surface attachment moieties attached to a spacer moiety as well as those not attached to a spacer moiety. In various embodiments, at least 20%, preferably at least 30 % of the surface attachment moieties are attached to spacer moieties. In various embodiments, this percentage may be in the range of 20 to 90% or 20 to 80% or 30 to 70% or 30 to 60%.

Similarly, not all spacer moieties need to be covalently attached to a charge optimization moiety. In various embodiments, at least part of the spacer moieties are not attached to a charge optimization moiety. This means that the surface functionalization comprises spacer moieties attached to a charge optimization moiety as well as those not attached to a charge optimization moiety. In various embodiments, at least 20%, preferably at least 30 % of the spacer moieties are attached to charge optimization moieties. In various embodiments, this percentage may be in the range of 20 to 90% or 20 to 80% or 30 to 70% or 30 to 60 or 30 to 50%.

In various embodiments, not all charge optimization moieties are covalently attached to a linker moiety. In various embodiments, at least part of the charge optimization moieties are not attached to a linker moiety. This means that the surface functionalization comprises charge optimization moieties attached to a linker moiety as well as those not attached to a linker moiety. In various embodiments, at least 10% but less than 80%, preferably at least 20 % but less than 60 %, more preferably 20 to 50 %, even more preferably 30 to 40 % of the charge optimization moieties are attached to linker moieties.

Not all linker moieties need to be covalently attached to a molecular probe. In various embodiments, at least part of the linker moieties are not attached to a molecular probe. This means that the surface functionalization comprises linker moieties attached to a molecular probe as well as those not attached to a molecular probe. In various embodiments, it is preferred that as many as possible of the linker moieties are attached to a molecular probe. This means that in various embodiments, at least 20%, preferably at least 30 %, at least 40 % or at least 50 % of the linker moieties are attached to molecular probes.

The surface-functionalized optical element can comprise additional elements such as a surface functionalization moiety or matrix silane as defined herein or one or more incomplete molecular probe linker constructs, as defined herein. "Incomplete molecular probe linker constructs" are meant to refer to constructs which are bound to the surface of the surface-functionalized optical element, but which do not contain all moieties of the complete molecular probe linker construct (e.g. because their reactive functionality has been quenched during preparation), i.e. which lack the molecular probe, or the molecular probe and the linker moiety, or the molecular probe, linker moiety and charge optimization moiety, or the molecular probe, linker moiety, charge optimization moiety and spacer moiety. Accordingly, the charge optimization constructs defined herein below are a specific type of incomplete molecular probe linker construct.

Said incomplete molecular probe linker constructs arise from incomplete coupling reactions or due to spatial constraints on the surface which prevent coupling to the respective reaction partner. In case of the charge optimization constructs, the reaction may be deliberately controlled such that not all of the charge optimization constructs are coupled to a linker construct. This ensures that the functionality of the charge optimization moieties, i.e. adjusting the overall charge of the surface, is maintained and not lost due to coupling.

According to various embodiments, the surface-functionalized optical element is an IR-transparent optical element, wherein at least a part of the surface of the IR-transparent optical element is bound to a molecular probe by a molecular probe linker construct as defined herein, wherein at least part of the surface of the IR-transparent optical element is bound to charge optimization constructs as defined herein, and optionally wherein at least a part of the surface of the IR-transparent optical element is covalently bound to a surface functionalization moieties and/or matrix silanes as defined herein, and optionally wherein at least a part of the surface of the IR-transparent optical element is covalently bound to a incomplete molecular probe linker constructs other than the charge optimization constructs as defined herein.

### IR-transparent optical element

The surface-functionalized optical element comprises an IR-transparent optical element. The IR-transparent optical element may be any optical element which is suitable for surface functionalization.

The IR-transparent optical element may be an optical element which is suitable for use in an optical biosensor. According to one embodiment, the IR-transparent optical element is an IR-transparent optical element which is suitable for attenuated total reflection (ATR) spectroscopy, transmission spectroscopy, or surface-enhanced infrared absorption (SEIRA) spectroscopy. According to one preferred embodiment, the IR-transparent optical element is suitable for attenuated total reflection infrared (ATR-IR) spectroscopy or transmission infrared spectroscopy.

The IR-transparent optical element is preferably at least transparent to infrared radiation with wavenumbers in a range of 40 to 13000 cm⁻¹, more preferably in the range of 500 to 4000 cm⁻¹, even more preferably in the range of 900 to 4000 cm⁻¹, like in the range of 1400 to 1800 cm⁻¹ or 1500 to 1750 cm⁻¹.

According to one preferred embodiment, the IR-transparent optical element is an IR-transparent optical waveguide, like an ATR-IR waveguide. According to one preferred embodiment, the IR-transparent optical element is an ATR-IR waveguide.

According to one embodiment, the IR-transparent optical element is a material and/or crystal selected from the group consisting of plastic, glass, diamond, germanium, silicon, silicon dioxide, silver halides, GaAs, ZnSe, ZnS, thallium(I) mixed halides (e.g. KRS-5), and AMTIRs. "AMTIR" is an amorphous material transmitting infrared radiation which is known in the art. A suitable AMTIR may be Ge₃₃As₁₂Se₅₅ (AMTIR-1).

According to one preferred embodiment, the IR-transparent optical element is an ATR-IR waveguide, which is a silicon crystal or a germanium crystal, and more preferably which is a silicon crystal.

The IR-transparent optical element may be at least partially surface-modified, e.g. by an oxide layer (e.g. silicon dioxide layer). The surface modification can further aid the surface-functionalization of the optical element, e.g. the attachment of a surface attachment moiety which is covalently bound to the optical element by a silane group.

Hence, in one embodiment of the invention, at least a part of the IR-transparent optical element has an oxide surface layer, and at least a part of the oxide surface is surface-functionalized. The oxide surface layer is preferably a metal oxide surface layer or a metalloid oxide surface layer. A "metalloid oxide" is an oxide of an element selected from the group consisting of boron, silicon, germanium, arsenic, antimony and tellurium.

More preferably, the metal oxide surface layer or a metalloid oxide surface layer is a silicon dioxide surface layer.

According to one embodiment, at least a part of the IR-transparent optical element has an oxide surface layer, preferably a silicon dioxide surface layer, wherein at least a part of the oxide surface layer is covalently bound to a silane group of the surface attachment moiety.

According to one preferred embodiment, the IR-transparent optical element is an attenuated total reflection infrared (ATR-IR) waveguide, and at least a part of the IR-transparent optical element has an oxide surface layer, and at least a part of the oxide surface layer is functionalized.

According to one preferred embodiment, the IR-transparent optical element is a silicon crystal or a germanium crystal, wherein the silicon crystal or the germanium crystal has an oxide surface layer, preferably a silicon dioxide surface layer, and wherein at least a part of the oxide surface layer is functionalized.

### Surface functionalization

At least a part of surface of the optical element is surface-functionalized, wherein said surface functionalization comprises a surface attachment moiety (S) covalently attached to the surface of the IR-transparent optical element, and wherein at least part of the surface attachment moieties is covalently attached to a spacer moiety (SP) comprising a poly(alkylene oxide), wherein at least part of the spacer moieties is covalently attached to a charge optimization moiety (CO), wherein said charge optimization moiety comprises a functional group that is charged under conditions of use of the optical element and optionally a poly(alkylene oxide), wherein at least part of the charge optimization moiety is covalently attached to a linker moiety (L) comprising a poly(alkylene oxide), wherein at least part of the linker moieties is covalently attached to a molecular probe.

"Covalently attached " in the meaning of the present disclosure can be understood in that the molecular probe linker construct comprises at least one continuous chain of sigma-bonds which connects the surface of the IR-transparent optical element to the molecular probe.

According to one embodiment, the surface functionalization does not contain a coordination transition metal complex, a protein-ligand complex, and/or a protein-protein complex. A coordination transition metal complex may be, but is not limited to, a histidine nickel complex. A protein-ligand complex may be, but is not limited to, a biotin-avidin or biotin-streptavidin complex. A protein-protein complex may be, but is not limited to, an antibody-antigen complex.

### Surface attachment moiety

The surface functionalization comprises a surface attachment moiety. The surface attachment moiety covalently attaches further moieties of the surface functionalization to the optical element. The surface attachment moiety is not particularly limited as long as the moiety is suitable for covalently attaching to the surface of the optical element.

According to one embodiment, the surface attachment moiety comprises a silane group or a sulfide group, and preferably a silane group.

For example, a silane group can be used for attaching the surface attachment moiety to an IR-transparent optical element (e.g. a silicon optical element) having an oxide surface layer such as a metalloid oxide surface layer (e.g. silicon dioxide).

A sulfide group can be used for attaching the surface attachment group to an IR-transparent optical element which is a germanium optical element and/or germanium crystal.

The silane group can have the formula of:

//-O-Si(R¹)₂-A,

wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
// represents the attachment to the surface of the IR-transparent optical element, and
A represents the attachment to the remaining construct.

According to one embodiment, the surface attachment moiety has a structure according to formula (SI):

//-Y¹-L¹-X¹-$$¹ (S-I),

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is an optional linking unit,
X¹ is a group selected from -C(O)NH-, -NHC(O)-, -NHC(O)HN-, and -NHC(O)O-,
$$¹ represents attachment to the spacer moiety.

According to one preferred embodiment, the surface attachment moiety has a structure according to formula (S-I):

//-Y¹-L¹-X¹-$$¹ (S-I),

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element, and optionally wherein each R¹ is independently selected from C₁-C₆-alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is a C₁-C₁₂ alkylene group, and preferably a C₁-C₆ alkylene group (e.g. propylene),
X¹ is a group selected from -C(O)NH- and -NHC(O)-, and preferably is -NHC(O)-,
$$¹ represents attachment to the spacer moiety.

In these embodiments, alkyl and alkoxy may be unsubstituted and may be selected from methyl, ethyl, methoxy and ethoxy, preferably methyl or methoxy, more preferably methyl.

It is understood that a linking group, such as L¹, needs to have two free valencies to be able to connect Y¹ and X¹. Alkylene as used herein above, refers to alkyl groups that have two free valencies, such as -(CH₂)₁₋₆-. In various embodiments, all linking groups or units referred to herein as L¹, L², L³, etc. are selected from such alkylene groups.

In various embodiments, the surface attachment moiety has the structure -O-Si(R¹)₂-(CH₂)₂₋₄-NHC(O)-, for example -O-Si(R¹)₂-(CH₂)₃-NHC(O)-, wherein R¹ is each independently methyl or -O-Si^{x}, preferably one R¹ is methyl and the other is -O-Si^{x} or both are methyl.

### Spacer moiety

Furthermore, the surface functionalization comprises a spacer moiety. The spacer moiety comprises an alkylene oxide (AO), such as (AO)ₙ₁ wherein n¹ is an integer from 1 to 50, such as 2 to 25, preferably from 2 to 10, more preferably from 2 to 8, most preferably from 3 to 5. n¹ may for example be 2, 3, 4, 5 or 6, such as 3, 4, or 5.

The alkylene subunit of the alkylene oxide can optionally be substituted. The alkylene oxide may be, but is not limited to, ethylene oxide, propylene oxide, butylene oxide, or block copolymers thereof.

Preferably, the alkylene oxide is poly(ethylene oxide), which is also referred to in the art as polyethylene glycol or PEG and abbreviated EO. According to one preferred embodiment, the alkylene oxide is a polyethylene glycol having 2 to 50 glycol repeating units, more preferably 2 to 30 repeating units, even more preferably 2 to 25 repeating units, yet even more preferably 2 to 16 repeating units, 3 to 8 repeating units or 3 to 6 repeating units or 3 to 5 repeating units. In various embodiments, it is (EO)₃ or (EO)₅.

The spacer moiety preferably further comprises a ligation group. The ligation group binds the spacer moiety to the charge optimization moiety. Preferably, the ligation group is covalently bound between the alkylene oxide of the spacer moiety and the charge optimization moiety.

The one or more ligation groups can be, but is not limited to, an ester, a hydrazone, an amide, a hydrazone, a succinimide or a ring-opened product of a succinimide, or a group which is obtainable by a bioorthogonal ligation reaction.

The skilled person knows bioorthogonal ligation reactions and the chemical structures of the groups which are obtainable by said reactions. Bioorthogonal ligation reactions include, but are not limited to, Staudinger ligations, [2+3]-cycloadditions between azides and alkynes ("click reaction), between azides and cycloalkynes ("copper-free click reaction"), between nitrones and cycloalkynes, [2+4]-cycloadditions between tetrazine and a trans-alkene ("tetrazine ligation"), oxime/hydrazine formation from aldehydes and ketones, and the like. Hence, the bioorthogonal ligation reaction is preferably one of the following reactions: (i) a reaction between an azide and a phosphine or phosphite; (ii) a reaction between an azide and an alkyne (including cycloalkynes); (iii) a reaction between an azide and a cycloalkyne; (iv) a reaction between a tetrazine and a trans-alkenes; (v) an oxime- and/or hydrazone-forming reaction.

For example, the ligation group can be an ester, a hydrazone, an amide, a succinimide, or a ring-opened product of a succinimide, which optionally has the one of the following structures: 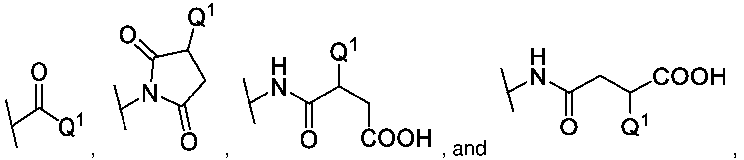 wherein Q¹ is a NH, O or S atom(s) of the charge optimization moiety.

In one embodiment, the spacer moiety comprises a ligation group which is obtainable by a bioorthogonal ligation reaction. According to one embodiment, the spacer moiety comprises a N-heterocyclic group, which is optionally selected from the group consisting of triazole groups, dihydropyridazine groups, a pyridazine groups, and isoxazoline groups.

According to one preferred embodiment, the spacer moiety comprises a N-heterocyclic group, which is selected from the group consisting of triazole groups, dihydropyridazine groups, a pyridazine groups, and isoxazoline groups. Preferably, the N-heterocyclic group is a triazole group. Preferably, the N-heterocyclic group is covalently bound between the poly(alkylene oxide) of the spacer moiety and the charge optimization moiety.

According to one embodiment, the spacer moiety comprises a ligation group which is an optionally substituted N-heterocyclic group having a structure according to any one of formula (HET-I) to (HET-VI): 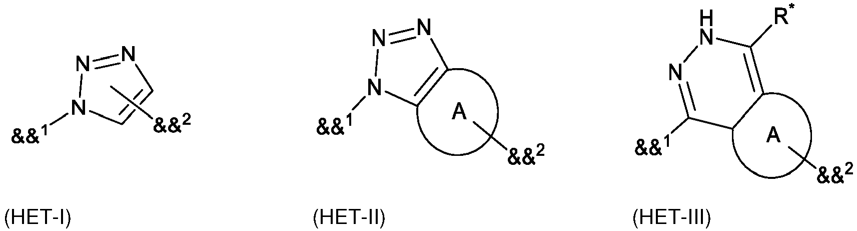 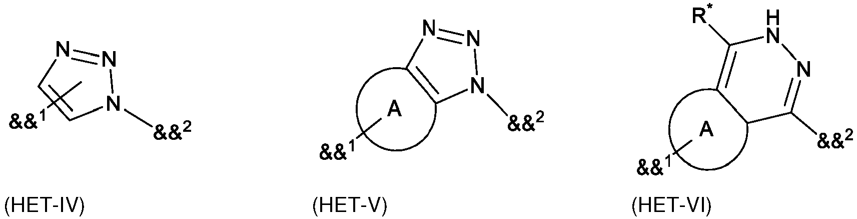 wherein
&&¹ represents attachment to a linking unit of the spacer moiety or to the poly(alkylene oxide),
&&² represents the attachment to a linking unit of the to the charge optimization moiety (CO), and
   ring A is an optionally substituted 8- to 9-membered mono-, bi- or multicyclic carbocycle or an optionally substituted 8- to 9-membered mono-, bi- or multicyclic heterocycle, and is preferably an optionally substituted dibenzo-fused 8-membered N-heterocycle or an optionally substituted bicyclo[6.1.0]non-4-ene ring, more preferably an optionally substituted bicyclo[6.1.0]non-4-ene ring; and
preferably the optionally substituted N-heterocyclic group has the structure according to formula (HET-II) or (HET-V):

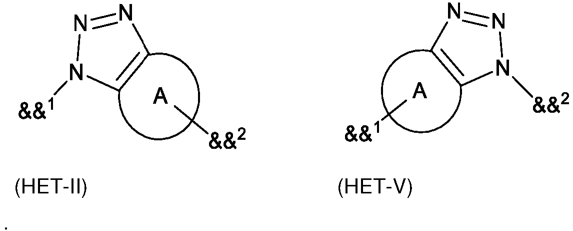

According to one embodiment, the spacer moiety has a structure according to formula (SP-II)

$$²-L²-X²-L³-H¹-&&¹ (SP-II),

wherein
$$² represents attachment to the surface attachment moiety,
L² to L³ are each independently an optional linking unit, for example an C₁-C₁₂ alkylene group, preferably a C₁-C₆ alkylene group;
X² is -(AO)n¹-, wherein AO is an alkylene oxide, preferably ethylene oxide (EO) or propylene oxide (PO), more preferably ethylene oxide, and n¹ is an integer from 2 to 25, preferably from 2 to 10, more preferably from 2 to 8, most preferably from 3 to 5,
H¹ is an optionally substituted N-heterocyclic group having a structure according to any one of formula (HET-I) to (HET-VI): 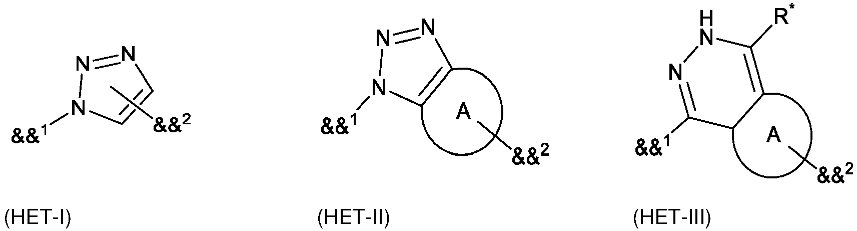 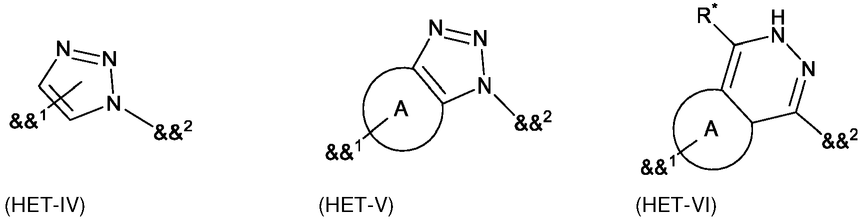 wherein
   &&¹ represents attachment to L³ or, if L³ is absent, corresponds to X²,
   &&² represents the attachment to the charge optimization moiety (CO),
   R* is H, an optionally substituted alkyl group or an optionally substituted aryl group, and preferably is a C₁-C₆ alkyl group (e.g. methyl);
   ring A is an optionally substituted 8- to 9-membered mono-, bi- or multicyclic carbocycle or an optionally substituted 8- to 9-membered mono-, bi- or multicyclic heterocycle, and is preferably an optionally substituted dibenzo-fused 8-membered N-heterocycle or an optionally substituted bicyclo[6.1.0]non-4-ene ring, more preferably an optionally substituted bicyclo[6.1.0]non-4-ene ring, and preferably the optionally substituted N-heterocyclic group has the structure according to formula (HET-II) or (HET-V):

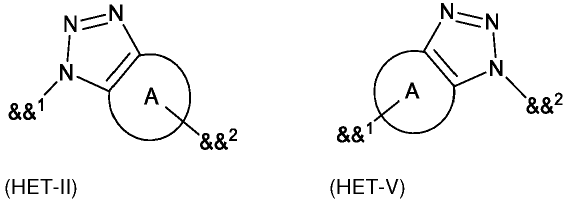

In various embodiments, the ligation moiety H¹ has a structure selected from: 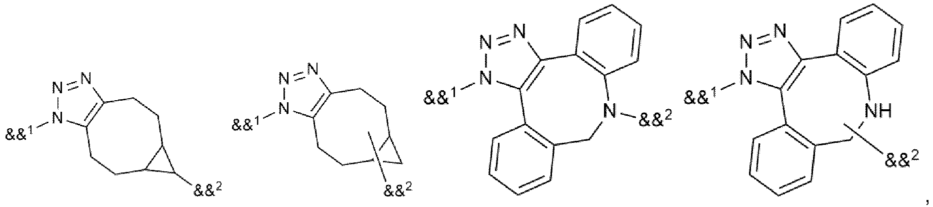 preferably

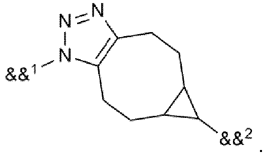

In various embodiments, one of L² and L³ is (CH₂)₁₋₃, such as (CH₂)₂ and the other is absent. "Optional" in relation to a linking unit, as used herein, means that it can be present or absent. X¹ can preferably be (EO)₃ or (EO)₅. H¹ is preferably HET-II, wherein A is an unsubstituted bicyclo[6.1.0]non-4-ene ring. In various embodiments, one of L² and L³ is (CH₂)₂ and the other is absent, X¹ is (EO)₃ or (EO)₅ and H¹ is HET-II, wherein A is an unsubstituted bicyclo[6.1.0]non-4-ene ring.

### Charge optimization moiety

Furthermore, the surface functionalization comprises a charge optimization moiety. The charge optimization moiety has been found to positively influence the properties of the surface, since it allows the adjustment of the net surface charge. Existing surface functionalizations suffer from the drawback that the surface of the sensor element has an inherent charge that stems from its oxide layer, such as silicon oxide, which form silanoles. In case of silicon oxide, this is typically a net negative charge. This is charge of the surface creates the issue that in addition to the intended target molecules that are to be captured and bound by the molecular probe, nonspecific adsorption to the surface occurs due to charge-mediated interaction with molecules in the sample. This is particularly problematic in case of complex samples, such as blood samples, which contain high amounts of positively charged proteins.

While existing blocking layers, such as casein-based blocking layers can reduce such nonspecific interaction to a certain extent, it has been found that this is still insufficient for highly sensitive detection of target analytes and that the blocking properties of the surface functionalization, in particular for proteins and especially positively charged proteins, can be significantly improved by adding charge optimization moieties to the layer which allow a fine control of the charge properties of the surface. An ideal surface has a neutral net charge but is zwitterionic in that it comprises negative as well as positively charged moieties. This allows rejection of positively as well as negatively charged proteins and other molecules present in the sample and thus significantly contributes to minimizing nonspecific adsorption, while not impairing the specific binding of the molecular probe to the target analyte. As mentioned above, it has been found that this works particularly well for complex samples containing proteins other than the analyte in high concentrations, such as blood samples.

The inventors have found that by introducing a charge optimization moiety, in particular a positively charged charge optimization moiety the nonspecific binding is significantly reduced relative to other known blocking layers used for the same purpose and under identical conditions.

The charge optimization moiety comprises a functional group that is charged under conditions of use of the optical element. "Charged under conditions of use of the optical element", as used herein, means that under measuring conditions, the respective functional group bears a net charge. Conditions that may influence the charge are, for example, pH conditions. The functional group bearing the charge is however selected such that it has the desired charge under measuring conditions, for example in a pH range of 5.5 to 9.0 or 6.0 to 8.5 or 6.5 to 7.5. Suitable functional groups are known to those skilled in the art. Suitable examples include, without limitation, amine groups, guanidine groups (-NH-C(=NH)-NH₂), carboxyl groups, sulfate groups and phosphate groups. In case a positive charge is desired, amine or guanidine groups are preferred, in particular primary amine groups that form -NH₃⁺ groups under measuring conditions. In various embodiments, the charged group is metal-free. In various embodiments, it may be an organic group or an amine group or a nitrogen-containing group. In various embodiments, it may have a molecular weight of 16 to 100 Da, such as 16 to 65 Da.

The functional group is at least charged, preferably positively charged, if it is not covalently attached to a linker moiety. The attachment of the linker moiety may make use of the charged moiety for the linking reaction and such lead to the loss of the charged group. In such embodiments, those charge optimization moieties covalently bound to a linker moiety lack the charged functional group. Accordingly, it is important that not all charge optimization moieties are covalently attached to a linker moiety. However, to allow analyte detection, at least part (but not all) of the charge optimization moieties are covalently attached to the linker moiety. In various embodiments, the charge optimization moiety therefore comprises a poly(alkylene oxide) and a functional group that is charged under conditions of use of the optical element if the charge optimization moiety is not covalently attached to a linker moiety.

In various embodiments, the number of the charge optimization moieties not covalently attached to the linker moiety is adjusted such that a specific net charge of the surface is achieved. In one embodiment the net charge to be achieved is about zero, wherein a net charge of about zero is understood by the person skilled in the art as about an equal number of positive charges and negative charges. Said control may be achieved by controlling the concentration of reaction partners, i.e. linker moiety forming molecules, in relation to the charge optimization moieties available for binding, i.e. the molar ratio of the two reaction partners. Such strategies are readily known to those skilled in the art and the charge properties may be determined by routine techniques. For example the individual steps of the build-up of the sensor element surface, i.e. the individual coupling steps, can be monitored in real-time by IR spectroscopy and thus can be stopped once the desired level of surface functionalization is reached.

As described above, the charge optimization moiety comprises a charged functional group, preferably an amine group, more preferably a primary amine group, at least as long as it is not covalently bound to a linker moiety.

If it is bound to a linker moiety, the originally charged group, such as the amino group or carboxyl group, may be converted into a group selected from -C(O)NH-, -NHC(O)-, -NHC(O)HN-, -NHC(O)O-, and -OC(O)NH.

The (originally) charged functional group is preferably a terminal group and linked to the rest of the molecule by a poly(alkylene oxide). The charge optimization moiety preferably also comprises a poly(alkylene oxide).

The poly(alkylene oxide) comprised may be of the formula (AO)ₙ₂ wherein n² is an integer from 1 to 50, such as 2 to 25, preferably from 2 to 10, more preferably from 2 to 8, most preferably from 2 to 5 or 2 to 4. n² may for example be 2, 3, 4, 5 or 6, such as 2, 3 or 4.

The alkylene subunit of the alkylene oxide can optionally be substituted. The alkylene oxide may be, but is not limited to, ethylene oxide, propylene oxide, butylene oxide, or block copolymers thereof.

Preferably, the alkylene oxide is poly(ethylene oxide), which is also referred to in the art as polyethylene glycol or PEG and abbreviated EO. According to one preferred embodiment, the alkylene oxide is a polyethylene glycol having 2 to 50 glycol repeating units, more preferably 2 to 30 repeating units, even more preferably 2 to 25 repeating units, yet even more preferably 2 to 16 repeating units, 2 to 8 repeating units or 2 to 6 repeating units or 2 to 5 or 2 to 4 repeating units. In various embodiments, it is (EO)₂, (EO)₃, or (EO)₄.

According to various embodiments, the charge optimization moiety has the structure according to formula (CO-I)

&&³-L⁴-X³-L⁵-X⁴-L⁶-C¹ (CO-I)

wherein
&&³ represents attachment to the spacer moiety (SP),
L⁴, L⁵ and L⁶ are each independently an optional linking unit,
X³ is a group selected from -C(O)NH-, -NHC(O)-, -NHC(O)HN-, -NHC(O)O-, and -OC(O)NH-,
X⁴ is absent or, preferably, is -(AO)ₙ₂-, wherein AO is an alkylene oxide, preferably ethylene oxide (EO) or propylene oxide (PO), more preferably ethylene oxide, and n² is an integer from 1 to 25, preferably from 2 to 10, more preferably from 2 to 5, most preferably 2 to 4,
C' is (1) a charged functional group, preferably an amine group, more preferably a primary amine group, or (2) has the structure

   X⁶-##¹
   wherein X⁶ is covalently attached to a L⁶ and is a group selected from -C(O)NH-, -NHC(O)-, -NHC(O)HN-, -NHC(O)O-, and -OC(O)NH-, and
   ##¹ represents attachment to the linker moiety (L).

L⁴, L⁵ and L⁶ are each independently an optional linking unit, as defined herein above. In various embodiments, they may, independently from each other, be absent or may be an C₁-C₁₂ alkylene group, preferably a C₁-C₆ alkylene group, for example CH₂ or CH₂-CH₂. L⁴ may preferably be methylene. In various embodiments, one of L⁵ and L⁶ is CH²-CH² and the other is absent.

X³ is, in various embodiments, -OC(O)NH-.

X⁴ is, in various embodiments, (EO)₂, (EO)₃, or (EO)₄.

C¹ is, in various embodiments where the charge optimization moiety is not attached to a linker moiety, NH₂. In these embodiments, C¹ of those charge optimization moieties attached to a linker moiety is -NHC(O)-##¹, -NHC(O)HN-##¹ or -NHC(O)O-##¹, preferably -NHC(O)-##¹.

In some embodiments, the charge optimization moiety has the structure of

-(CH₂)₁₋₂-OC(O)-NH-(CH₂CH₂O)₂₋₄-(CH₂)₂₋₄-NH₃⁺,

or

-(CH₂)₁₋₂-OC(O)-NH-(CH₂CH₂O)₂₋₄-(CH₂)₂₋₄-NHC(O)-##¹.

wherein
##¹ represents attachment to the linker moiety (L).

Preferably the charge optimization moiety has the structure of

-(CH₂)-OC(O)-NH-(CH₂CH₂O)₂-(CH₂)₂-NH₃⁺,

or

-(CH₂)-OC(O)-NH-(CH₂CH₂O)₂-(CH₂)₂-NHC(O)-##¹,

wherein
##¹ represents the attachment to the linker moiety.

### Linker moiety

The surface functionalization further comprises a linker moiety. The linker moiety is intended to link the molecular probe to the surface. The linker moiety comprises at least one alkylene oxide (AO) unit, but may comprise two or more alkylene oxide units.

The alkylene subunits of the poly(alkylene oxide) can optionally be substituted. The poly(alkylene oxide) may be, but is not limited to, poly(ethylene oxide), poly(propylene oxide), poly(butylene oxide), or block copolymers thereof.

Preferably, the poly(alkylene oxide) is poly(ethylene oxide), which is also referred to in the art as polyethylene glycol or PEG. According to one preferred embodiment, the alkylene oxide is a polyethylene glycol having 1 to 50 repeating units, more preferably 2 to 25 repeating units, even more preferably 2 to 15 repeating units, yet even more preferably 3 to 13 or 4 to 8 or 8 to 13 repeating units. As described above, the linker moiety may comprise two or more poly(alkylene oxide) groups within the molecule, as defined herein below. In these instances, each of the groups comprises 3 to 13 or 4 to 8 or 8 to 13 units.

The linker moiety preferably further comprises a ligation group. The ligation group binds the molecular probe to a linking unit of the linker moiety, or to another moiety of the linker construct. Preferably, the ligation group is covalently bound between the poly(alkylene oxide) of the linker and the molecular probe.

The ligation group can be understood as a structural unit which is obtained when preparing the surface functionalization, and particularly when (i) reacting precursors of the moieties of the surface functionalization with one another, (ii) attaching the molecular probe to the linker moiety of the surface functionalization, or (iii) reacting a precursor of the moieties of the surface functionalization with a molecular probe which is pre-labeled with a precursor of the linker moiety.

Depending on the structure of the linker construct and its preparation process, the linker moiety can comprise one or more ligation groups.

The one or more ligation groups can be, but is not limited to, an ester, a hydrazone, an amide, a hydrazone, a succinimide or a ring-opened product of a succinimide, or a group which is obtainable by a bioorthogonal ligation reaction.

The skilled person knows bioorthogonal ligation reactions and the chemical structures of the groups which are obtainable by said reactions. Bioorthogonal ligation reactions include, but are not limited to, Staudinger ligations, [2+3]-cycloadditions between azides and alkynes ("click reaction), between azides and cycloalkynes ("copper-free click reaction"), between nitrones and cycloalkynes, [2+4]-cycloadditions between tetrazine and a trans-alkene ("tetrazine ligation"), oxime/hydrazine formation from aldehydes and ketones, and the like. Hence, the bioorthogonal ligation reaction is preferably one of the following reactions: (i) a reaction between an azide and a phosphine or phosphite; (ii) a reaction between an azide and an alkyne (including cycloalkynes); (iii) a reaction between an azide and a cycloalkyne; (iv) a reaction between a tetrazine and a trans-alkenes; (v) an oxime- and/or hydrazone-forming reaction.

In one embodiment, the linker moiety comprises a ligation group which is covalently bound to the molecular probe. For example, the ligation group can be an ester, a hydrazone, an amide, a succinimide, or a ring-opened product of a succinimide, which optionally has the one of the following structures: 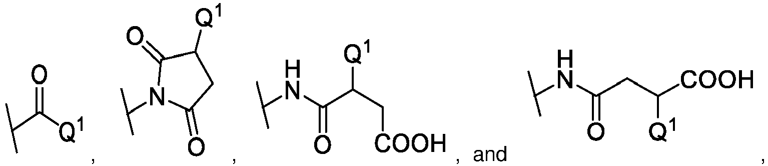 wherein Q¹ wherein Q¹ is a NH, O or S atom(s) of the molecular probe. If the molecular probe contains amino acids (e.g. when the molecular as an antibody), Q¹ may be NH, O or S of an amino acid of the molecular probe. For example, Q¹ may be an NH of a lysine side chain.

In one embodiment, the linker moiety comprises a ligation group which is obtainable by a bioorthogonal ligation reaction. According to one embodiment, the linker moiety comprises a N-heterocyclic group, which is optionally selected from the group consisting of triazole groups, dihydropyridazine groups, a pyridazine groups, and isoxazoline groups.

According to one preferred embodiment, the linker moiety comprises a N-heterocyclic group, which is selected from the group consisting of triazole groups, dihydropyridazine groups, a pyridazine groups, and isoxazoline groups. Preferably, the N-heterocyclic group is a triazole group. Preferably, the N-heterocyclic group is covalently bound between the poly(alkylene oxide) of the linker moiety and the molecular probe.

According to one embodiment, the linker moiety comprises a ligation group which is an optionally substituted N-heterocyclic group having a structure according to any one of formula (HET-I) to (HET-VI): 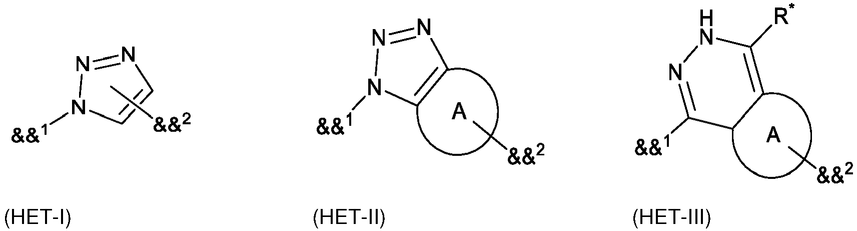 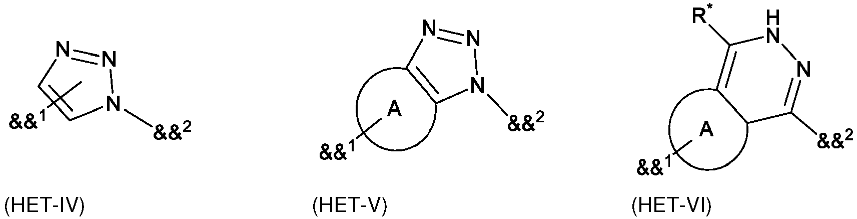 wherein
&&¹ represents attachment to the rest of the linker moiety,
&&² represents the attachment to the molecular probe.

In various embodiments, the linker moiety has the structure according to formula (L-I) or (L-II)

##²-L⁷-X⁷-L¹⁰-H²-L¹¹-** (L-I),

or

(##²-L⁷-X⁷-L⁸)₂X⁸-L⁹-X⁹-L¹⁰- H²-L¹¹-** (L-II)

wherein
each ##² represents attachment to the charge optimization moiety (CO),
L⁷, L⁸, L⁹, L¹⁰ and L¹¹ are each independently an optional linking unit, for example an C₁-C₁₂ alkylene group, preferably a C₁-C₆ alkylene group, for example CH₂ or CH₂-CH₂
X⁷ is -(AO)ₙ₃-, wherein AO is an alkylene oxide, preferably ethylene oxide (EO) or propylene oxide (PO), more preferably ethylene oxide, and n³ is an integer from 1 to 25, preferably from 2 to 10, more preferably from 3 to 9,
X⁸ is NC(O)-, NC(O)NH- or NC(O)O-,
X⁹ is -(AO)ₙ₄-, wherein AO is an alkylene oxide, preferably ethylene oxide (EO) or propylene oxide (PO), more preferably ethylene oxide, and n⁴ is an integer from 1 to 25, preferably from 2 to 10, more preferably from 2 to 5, most preferably 2 to 4,
wherein the sum of all n³ and n⁴ is preferably 4 to 25, more preferably 5 to 14,
H² is an optionally substituted N-heterocyclic group having a structure according to any one of formulae (HET-I) to (HET-VI): 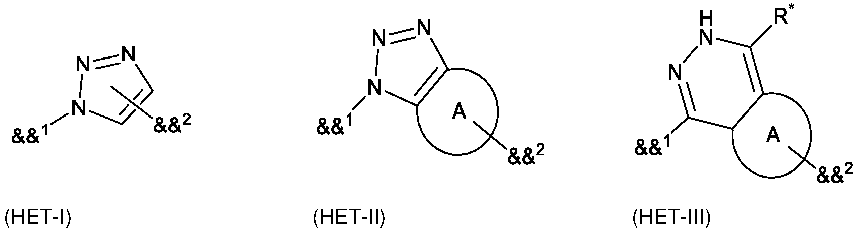 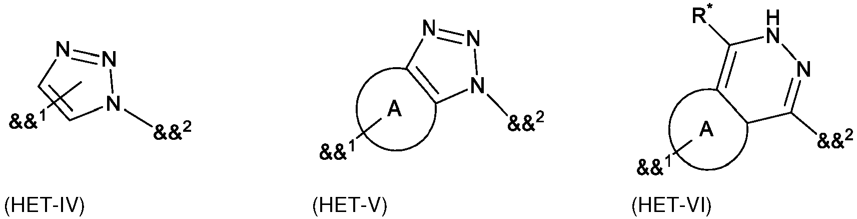 wherein
   &&¹ represents attachment to L¹⁰ or, if L¹⁰ is absent, to X⁹,
   &&² represents the attachment to L¹¹ or, if L¹¹ is absent, corresponds to **,
   R* is H, an optionally substituted alkyl group or an optionally substituted aryl group, and preferably is a C₁-C₆ alkyl group,
   ring A is an optionally substituted 8- to 9-membered mono-, bi- or multicyclic carbocycle or an optionally substituted 8- to 9-membered mono-, bi- or multicyclic heterocycle, and is preferably an optionally substituted dibenzo-fused 8-membered N-heterocycle or an optionally substituted bicyclo[6.1.0]non-4-ene ring, more preferably an optionally substituted dibenzo-fused 8-membered N-heterocycle, and
   ** represents the attachment to another moiety of the molecular probe linker construct or to the molecular probe.

In various embodiments, the optionally substituted N-heterocyclic group has the structure according to formula (HET-II) or (HET-V):

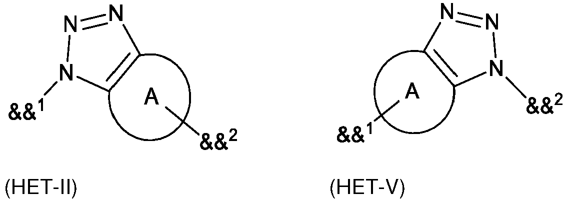

In various embodiments, H² may be selected from: 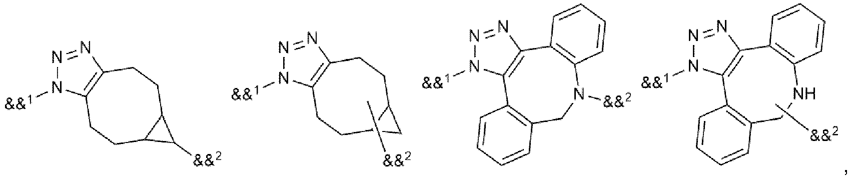 in particular

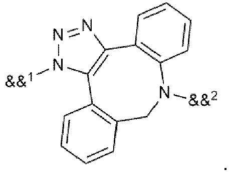

In various embodiments, L⁷ is CH₂ or CH₂-CH₂, preferably ethylene.

In various embodiments, where the linker moiety has the structure ##²-L⁷-X⁷-L¹⁰-H²-L¹¹-**, X⁷ is (EO)₅₋₁₀, preferably (EO)₇₋₉, more preferably (EO)₈. In such embodiments, L¹⁰ may be absent. In some embodiments, the linker moiety has the structure ##²-(CH₂)₂-(OCH₂CH₂)₈-H²-L¹¹-**, wherein H² and L¹¹ are as defined above, preferably H² is HET-II.

In various embodiments, where the linker moiety has the structure (##²-L⁷-X⁷-L⁸)₂X⁸-L⁹-X⁹-L¹⁰-H²-L¹¹-**, each X⁷ and X⁸ is independently (EO)₃₋₈, preferably (EO)₃₋₆, more preferably (EO)₄. In such embodiments, X⁸ is NC(O)-, NC(O)NH- or NC(O)O-, preferably NC(O)- and L⁸ is absent. It is understood that since X⁸ is linked to three other parts of the molecule, in these groups the N atom has two free valencies that connect to L⁸ or, if it is absent, X⁷. L⁹ may be CH₂-CH₂. L¹⁰ may be absent. In some embodiments, the linker moiety has the structure (##²-(CH₂)₂-(OCH₂CH₂)₄)₂NC(O)(CH₂)₂-(OCH₂CH₂)₄-H²-L¹¹-**, wherein H² and L¹¹ are as defined above, preferably H² is HET-II.

### Structures of the charge optimization construct and the molecular probe linker construct

The molecular probe linker construct can have different chemical structures depending on the synthetic procedures for attaching the molecular probe to the surface of the IR-transparent optical element, and depending on the number of moieties of the linker construct.

The surface functionalization can generally be defined by the structure according to formula (A), which is referred to herein as the charge optimization construct, and the structure according to formula (B), which is referred to herein as the molecular probe linker construct:

//-(S)-(SP)-(CO) (A)

//-(S)-(SP)-(CO)-(L)-\\ (B)

wherein
// represents attachment to the surface of the IR-transparent optical element,
(S) represents the surface attachment moiety,
(SP) represents the spacer moiety,
(CO) represents the charge optimization moiety,
(L) represent the linker moiety, and
\\ represents attachment to another moiety of the molecular probe linker construct or to the molecular probe.

Both the molecular probe linker construct and the charge optimization construct may optionally comprise additional moieties which are bound between the spacer moiety and charge optimization moiety as well as between the charge optimization moiety and the linker moiety and between the linker moiety and the molecular probe.

Further embodiments of suitable surface attachment moieties, spacer moieties and linker moieties are disclosed in WO2024003214A2 and WO2024003213A1, which are incorporated by reference in their entirety. More specifically, surface attachment moieties are disclosed at pages 5-7 and 13-14 and spacer moieties at pages 7-11 and 14-20 of WO2024003214A2. In various embodiments, the surface functionalization as described herein does not comprise a peptide moiety as disclosed in WO2024003214A2 or a peptide compound as disclosed in WO2024003213A1 .

### Molecular probe

The surface-functionalized optical element comprises a molecular probe.

The molecular probe can be attached to the linker moiety by a ligation group. The ligation group can be, but is not limited to, an ester, a hydrazone, an amide, a hydrazone, a succinimide or a ring-opened product of a succinimide, or a group which is obtainable by a bioorthogonal ligation reaction (e.g. a N-heterocyclic group as defined herein above).

Preferably, the molecular probe is covalently bound to the linker construct by a group which is obtainable by a bioorthogonal ligation reaction, or by an ester, or an amide, or a succinimide or a ring-opened product thereof.

For example, the molecular probe can be covalently bound to the linker construct by one of the following structures: 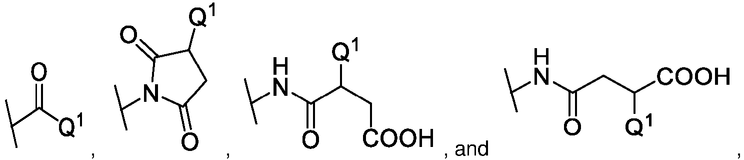 wherein Q¹ is a NH, O or S atom(s) of the molecular probe, and "\" represents the attachment of the remaining linker construct. Preferably, the molecular probe is bound to the linker construct by the following group: : 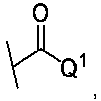 wherein Q¹ is a NH or O atom of the molecular probe, and "\" represents the attachment of the remaining linker construct

The molecular probe is not particularly limited and may be selected by the person of skill according to the target analyte which is to be detected and/or analyzed, and/or the target chemical interaction which is to be detected or analyzed. For example, the molecular probe can be a molecular probe which is known in the art to be useful in ATR-IR-spectroscopy and biosensors using said spectroscopy.

According to one embodiment, the molecular probe is an antigen, an antibody, a fragment of an antibody, an antibody fusion protein or a fusion protein with an antibody fragment, an antibody conjugate or a conjugate with an antibody fragment, a protein complex which optionally contains an antibody fragment or a fusion protein thereof, an anticalin, a nanobody, a small organic molecule, a drug, a nucleic acid, an aptamer, a lipid, a carbohydrate, a peptide, or mixtures thereof.

According to one embodiment, the molecular probe is an antigen-binding protein, optionally selected from an antibody, a fragment of an antibody, an antibody fusion protein or a fusion protein with an antibody fragment, an antibody conjugate or a conjugate with an antibody fragment, or a protein complex which optionally contains an antibody fragment or a fusion protein thereof, an anticalin, a nanobody, and mixtures thereof. In various embodiments, the antibody fragment also includes single chain variable fragments (scFv).

According to one preferred embodiment, the molecular probe is an anti-amyloid β antibody, an anti-alpha synuclein antibody, an anti-tau antibody, an anti-TDP-43 antibody, or a fragment, a fusion protein and/or a conjugate of said antibodies.

According to one embodiment, the molecular probe is an anti-amyloid β antibody, or a fragment, a fusion protein, and/or an conjugate thereof.

According to one embodiment, the molecular probe is an anti-alpha synuclein antibody, or a fragment, a fusion protein, and/or an conjugate thereof. The anti-alpha synuclein antibody may be a single-chain variable fragment (scFv), for example an scFv comprising the heavy chain variable domain having the amino acid sequence of SEQ ID NO:8 and the light chain variable domain having the amino acid sequence of SEQ ID NO:9. In various embodiments, such scFv comprises, from N- to C-terminus, the amino acid sequence: SEQ ID NO:8-SEQ ID NO:12-SEQ ID NO:9, covalently linked to each other by peptide bonds. In some embodiments, said scFv comprises or consists of the amino acid sequence of SEQ ID NO:10.

According to one embodiment, the molecular probe is capable of forming a complex with a protein which is associated with or may cause a protein misfolding disease. Protein misfolding diseases, also referred to in the art as proteopathies, are a class of disease in which one or more proteins are misfolded and/or become structurally abnormal, which can disrupt the function of cells, tissues, and/or organs. Protein misfolding diseases and proteins associated with protein misfolding diseases are known to the skilled person and include, but are not limited to, amyloid beta-peptide, tau proteins, alpha synuclein, TDP-43, and Huntingtin.

According to one preferred embodiment, the molecular probe is capable of forming a complex with a target analyte selected from the group consisting of amyloid-beta (Aβ) peptides and isoforms thereof, alpha synuclein, tau protein, TDP-43, human islet amyloid polypeptide (hiAPP), prion protein, and or p53.

The amyloid-beta (Aβ) peptides may be monomeric, oligomeric or fibrillar amyloid-beta (Aβ) peptides. Similarly, alpha synuclein may be monomeric, oligomeric or fibrillary.

If the molecular probe comprises an antibody or antibody fragment as defined herein above, the antibody may comprise an antibody labeling moiety which upon reaction with the linker moiety attached the surface forms the ligation group. Said antibody labeling moieties need one reactive group for covalent coupling to the antibody and a second reactive group, typically a different group, for reacting with the linker moiety attached to the sensor element surface. Examples for such moieties are described in the examples herein below and include, for example, (2,5-dioxopyrrolidin-1-yl 1-azido-3,6,9,12,15,18,21,24-octaoxaheptacosan-27-oate.

This similarly applies to all other types of molecular probes, which may be prepared by reacting the actual target binding part with an attachment moiety (or antibody labeling moiety) to form the molecular probe, where the latter serves the purpose to facilitate coupling to the sensor surface.

### Matrix silane

The surface-functionalized optical element can further comprise a surface functionalization moiety (SFM), wherein at least a part of the surface of the IR-transparent optical element is covalently bound to the surface functionalization moiety.

According to one embodiment, the surface functionalization moiety comprises a poly(alkylene oxide) group, preferably a poly(ethylene glycol) group, and an inert terminal group. An inert terminal group can be, but is not limited to, a terminal alkyl group such as a terminal C₁-C₆ alkyl group (e.g. methyl), or a terminal alkoxy group such as a terminal C₁-C₆ alkoxy group (e.g. methoxy).

According to one preferred embodiment, at least a part of the surface of the optical element is covalently bound to a matrix silane, wherein the matrix silane has the structure

//-Y¹-L¹-X¹-L^{1a}-X^{1a}-M¹

wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of another matrix silane being attached to the surface of the IR-transparent optical element,
L¹ and L^{1a} are each independently an optional linking unit,
X¹ is a group selected from -C(O)NH-, -NHC(O)-, -NHC(O)HN-, and -NHC(O)O-,
X^{1a} is -(AO)ₙ₁ₐ-, wherein AO is an alkylene oxide, preferably ethylene oxide (EO) or propylene oxide (PO), more preferably ethylene oxide, and n^{1a} is an integer from 2 to 25, preferably from 2 to 10, more preferably from 2 to 8, most preferably from 2 to 5 or 2 to 4, and
M¹ is a C₁-C₆ alkyl group, preferably methyl.

L¹ and L^{1a} are linking groups as defined herein above and may independently be C₁-C₁₂ or C₁-C₆ alkylene groups, such as -(CH₂)₁₋₆-.

The alkylene subunit of the poly(alkylene oxide) can optionally be substituted. The poly(alkylene oxide) may be, but is not limited to, poly(ethylene oxide), poly(propylene oxide), poly(butylene oxide), or block copolymers thereof. Preferably, the poly(alkylene oxide) is poly(ethylene oxide). According to one preferred embodiment, the poly(alkylene oxide) is a polyethylene glycol having 2 to 25 repeating units, more preferably 2 to 15 repeating units, even more preferably 2 to 10 repeating units, yet even more preferably 2 to 10 repeating units, for example 2 to 8 repeating units or 2 to 5 repeating units.

In various embodiments, the matrix silane has the structure -O-Si(R¹)₂-(CH₂)₂₋₄-NHC(O)-(CH₂CH₂O)₂-₄-(C₁-C₆) alkyl, wherein R¹ is each independently methyl or -O-Si^{x}, preferably one R¹ is methyl and the other is -O-Si^{x} or both are methyl, and wherein C₁-C₆ alkyl is preferably methyl.

The matrix silane can function as a matrix on the surface of the IR-transparent optical element which reduces nonspecific binding of media or sample components to the surface, and which further contributes to the stability of the surface-functionalization. Due to the presence of the unpolar alkyl head groups and the poly(alkylene oxide) part, it serves a blocking function and prevents nonspecific interaction of charged or polar molecules with the surface. Furthermore, the use of a matrix silane allows for fine tuning the amount of linker construct to be bound to the surface of the optical element and allows for spacing the molecular probe linker construct and the charge optimization construct on the surface of the optical element. The spacing of the molecular probe linker construct in turn allows for spacing the molecular probe on the surface of the optical element, which can improve interaction and/or detection between the molecular probe and the target analyte, e.g. between an antibody as molecular probe and a target antigen.

### Process for preparing the surface-functionalized optical element

In one further aspect, the invention provides a process for preparing a surface-functionalized optical element, and preferably of the surface-functionalized optical element according to the invention. The process comprises the steps of:
(a) providing an IR-transparent optical element,
(b) reacting at least a part of the surface of the IR-transparent optical element with a first compound to form an optical element surface-functionalized with a surface attachment moiety, wherein said surface attachment moiety comprises a reactive group,
(c) reacting the optical element surface-functionalized with a surface attachment moiety comprising a reactive group with a second compound to form an optical element surface-functionalized with a surface attachment moiety and a spacer moiety covalently attached to the surface attachment moiety, wherein the spacer moiety comprises a reactive group,
(d) reacting the optical element surface-functionalized with a surface attachment moiety and a spacer moiety comprising a reactive group with a third compound to form an optical element surface-functionalized with a surface attachment moiety, a spacer moiety and a charge optimization moiety covalently attached to the spacer moiety, wherein the charge optimization moiety comprises a charged functional group,
(e) reacting the optical element surface-functionalized with a surface attachment moiety, a spacer moiety and a charge optimization moiety comprising a charged functional group with a fourth compound to form an optical element surface-functionalized with a surface attachment moiety, a spacer moiety, a charge optimization moiety and a linker moiety covalently attached to the charge optimization moiety, wherein the linker moiety comprises a reactive group,
(f) reacting the optical element surface-functionalized with a surface attachment moiety, a spacer moiety, a charge optimization moiety and a linker moiety comprising a reactive group with a molecular probe to form an optical element surface-functionalized with a surface attachment moiety, a spacer moiety, a charge optimization moiety, a linker moiety and a molecular probe.

In step a), an IR-transparent optical element is provided. The IR-transparent optical element is preferably an IR-transparent optical element as defined herein above in connection with the surface-functionalized optical element of the invention.

In step b), at least a part of the surface of the IR-transparent optical element is reacted with a molecule that is a precursor of the surface attachment moiety. In step b), said precursor of the surface attachment moiety reacts with the surface of the IR-transparent optical element to create a covalent bond.

In preferred embodiments, the precursor of the surface attachment moiety is a silane. The silane group of the precursor reacts with the surface of the IR-transparent optical element to create a covalent bond and thus attach the surface attachment moiety to the surface. In order to allow further reactions, the precursor of the surface attachment moiety comprises a reactive group in addition to the groups used for covalent bonding to the surface, i.e. the silane group. Said reactive group may be any suitable group, for example one of the groups for bioorthogonal ligation disclosed herein. In various embodiments, it may be an amine group, which is then reacted with, for example, a carboxylic acid group to form an amide bond.

In step c), at least a part of the optical element surface-functionalized with a surface attachment moiety comprising a reactive group is reacted with a precursor molecule of the spacer moiety. In step c), the precursor of the spacer moiety reacts with the reactive group of the surface attachment moiety to create a covalent bond. To allow such a reaction, the precursor of the spacer moiety comprises at least one reactive group that can react with the reactive group of the surface attachment moiety and another reactive group to facilitate its later reaction with the charge optimization moiety precursor in step d). The reactive group for reacting with the surface attachment moiety may be a carboxylic acid group, as described above. Alternatively, all other options described herein for the ligation reaction may be used.

The precursor of the spacer moiety further comprises a reactive group intended to facilitate covalent attachment of the charge optimization moiety precursor in the next step. Said reactive group may again be any one of the groups disclosed for ligation reactions, in particular bioorthogonal ligation, herein above, such as an azide group.

In step d), at least a part of the spacer moieties covalently attached to surface attachment moieties are reacted with a charge optimization moiety precursor. In step d), the charge optimization moiety precursor reacts with the reactive group of the spacer moiety to create a covalent bond. To facilitate this reaction, said charge optimization moiety comprises a functional group different from the functional group that is at least charged, preferably positively charged, if said charge optimization moiety is not covalently attached to a linker moiety. Said reactive group may again be any one of the groups disclosed for ligation reactions, in particular bioorthogonal ligation, herein above, such as an alkyne group, in particular a cycloalkyne group. This then reacts with the azide group of the spacer moiety to form heterocyclic ligation unit, as disclosed herein above (such as H¹).

In step e), at least a part of the charge optimization moieties attached to the surface via the surface attachment moiety and the spacer moiety are reacted with a linker moiety precursor. In said step, the number of the charge optimization moieties not covalently attached to a linker moiety may be advantageously adjusted such that the net charge of the surface of the surface-functionalized optical element is about zero. As the silicon oxide surface is predominantly negatively charged and the charged functional group is typically lost by reaction with the linker moiety precursor, a sufficient number of the charge optimization moieties need to remain unreacted to provide for the desired charge balance. This may be controlled by the ratio of added linker moiety precursor, the reaction conditions or the like.

In step e), the linker moiety precursor reacts with the charged functional group of the charge optimization moiety to create a covalent bond and thus attach the linker moiety to the surface.

The term "precursor", as used in this context, is intended to refer to derivatives of the moieties described herein above that are not yet attached to the surface and thus comprise at least two reactive groups, one to allow covalent attachment to the surface or any moiety already bound thereto and another one to allow covalent coupling of further moieties. Once the precursors have been covalently attached and optionally also reacted with a further moiety to be attached to the surface they form the moieties described herein above, i.e. surface attachment moiety, spacer moiety, charge optimization moiety, and linker moiety. It is understood that while the above description describes these moieties, with the exception of the charge optimization moiety of which part remains deliberately unreacted, as being bound to at least two binding partners, all these moieties may in embodiments where their reaction is incomplete also comprise a free reactive group. Such incomplete molecular probe linker constructs and incomplete charge optimization constructs have also been described in more detail above.

In step f), at least part of the linker moieties attached to the surface are reacted with a molecular probe. In step f), the molecular probe is therefore covalently attached to the surface by its reaction with the reactive group of the linker moiety.

It is understood that between all the above steps, there may be one or more washing steps to remove unbound or unreacted molecules from the surface.

In various embodiments, it may be preferred that the surface of the optical element is first pre-functionalized, steps (b) to (e) followed by subsequent attachment of the molecular probe (step (f) onto the pre-functionalized surface. The process according to the invention can therefore be defined by the synthetic procedures covering the pre-functionalization and different procedures for attachment of the molecular probe.

Some of these methods relate to procedures, in which the surface of the IR-transparent optical element is at least partially pre-functionalized by a group selected from nucleophile-reactive groups, which is part of the linker moiety precursor and is not used for covalently attaching the linker moiety to the surface. Said nucleophile-reactive group may be suitable for directly attaching a molecular probe. Such groups include, without limitation, aldehydes, carboxylic acids, esters, active esters and maleimide, for example a NHS(N-hydroxy succinimide)-ester group. These groups may then in procedure (B) react with a nucleophilic group of the molecular probe.

For example, in case the molecular probe comprises one or more amine groups, e.g. as part of one or more lysine groups of a peptide/protein, the molecular probe can be reacted with a surface of the optical element which has been pre-functionalized such that a nucleophile-reactive group (e.g. a NHS-ester group) is present on its surface.

In other embodiments, the methods comprise procedures, in which the surface of the IR-transparent optical element is at least partially pre-functionalized by a group selected from bioorthogonal-reactive groups. "Bioorthogonal-reactive groups" are known to the skilled person and can be, but are not limited to, alkynes, strained cycloalkynes, strained cycloalkenes, azides, tetrazines, nitrones, norbornenes, nitrile oxides, oxanorbornadienes, and tetrazoles. "Strained cycloalkynes" are known to the skilled person and include, but are not limited to, 8-membered cycloalkynes. "Strained cycloalkenes" include, but are not limited to, trans-cyclooctene.

In such synthetic procedures the surface-functionalization comprises a bioorthogonal-reactive group which is suitable for attaching a linking unit of the linker construct, including, but not limited to, alkynes, strained cycloalkynes, strained cycloalkenes, azides, and tetrazines.

Accordingly, in various embodiments, step e) comprises reacting at least a part of the surface of the optical element with a linker moiety precursor comprising a ligation group X^{a} for attachment of the molecular probe.

X^{a} may be -N₃, a cyclo-trans-alkene group, an alkyne group, or 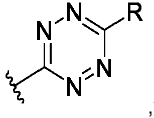 wherein R is an optional substituent, preferably an optional C₁-C₆ alkyl substituent (e.g. methyl), and preferably X^{a} is - N₃. The cyclo-trans-alkene group may be, but is not limited to, an optionally substituted 8-membered cyclo-trans-alkene optionally comprising one or more heteroatoms. Suitable alkyne groups of substituent X^{a} are alkyne groups comprising a structural unit of DBCO, DIBO, DIFO, or BCN (bicyclo[6.1.0]non-4-yne). These abbreviations are well-known in the art. For illustration, DBCO is 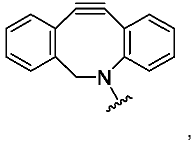 DIBO is 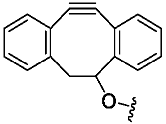 and DIFO is

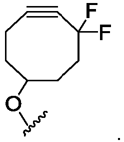

The pre-functionalized optical element can subsequently be reacted in a step c) with a molecular probe or a pre-labeled molecular probe to obtain the inventive surface-functionalized optical element.

The pre-functionalized optical element obtained in step (e) is subsequently reacted in a step (f) with a molecular probe which comprises one or more bioorthogonal reactive groups, e.g. an azide as part of an unnatural amino acid of a peptide/protein. In step (f), the surface-functionalized optical element according to the invention is obtained.

To facilitate this reaction, the molecular probe reacted in step (f) comprises one or more bioorthogonal reactive groups which are suitable for reacting with a bioorthogonal reactive group of the pre-functionalized surface of the optical element. Such pairs of bioorthogonal reactive groups are known to the skilled person and include, but are not limited to, azides/alkynes, azides/strained alkynes, trans-alkenes/tetrazenes.

The molecular probe may be pre-labeled with such a bioorthogonal reactive-group which is suitable for reacting with a bioorthogonal reactive group of the pre-functionalized surface of the optical element. Such pairs of bioorthogonal reactive groups are known to the skilled person and include, but are not limited to, azides/alkynes, azides/strained alkynes, trans-alkenes/tetrazenes.

The bioorthogonal reactive group can be covalently attached to the molecular probe by a linking unit. For example, the pre-labeling of the molecular probe may be carried out by reacting the molecular probe with a labeling compound comprising (i) a bioorthogonal reactive group, (ii) a nucleophile reactive group (e.g. NHS active ester), and wherein the nucleophile reactive group reacts with the molecular probe.

According to one embodiment, the pre-labeled molecular probe comprises an alkyne group, and optionally an alkyne group comprising a structural unit of DBCO, DIBO, DIFO, or BCN, in particular DBCO. In such embodiments, the surface-functionalization typically comprises a reactive azide group.

### Method for detecting a target analyte

In another aspect of the invention, a method for detecting a biomarker is provided. The method comprises the steps of
(i) bringing into contact a surface-functionalized optical element of the invention with a sample suspected to comprise a target analyte, and optionally wherein the sample is a human body fluid;
(ii) detecting the biomarker based on an interaction between the molecular probe and the target analyte, and optionally wherein the interaction between the molecular probe and the target analyte is detected by infrared spectroscopy, and optionally wherein the biomarker is based on an amide I band position of the target analyte.

The sample may be a body fluid such as, but not limited to, blood plasma or cerebrospinal fluid (CSF) sample. According to one embodiment, the sample is a blood plasma or CSF sample. The sample may be brought into contact with the surface-functionalized optical element in a flow chamber. In various embodiments, the sample is a blood sample or blood plasma or blood serum sample. The particular design of the surface-functionalization of the present invention allows the analysis of complex samples, such as blood (plasma or serum) samples, which was previously hampered by nonspecific binding and the resultant noise signal. The specific setup of the functionalization with a charge optimization unit allows a significant reduction of the nonspecific adsorption compared to other known blocking solutions, in particular those using peptides.

According to various embodiments, the target analyte is a peptide, polypeptide or protein. In various embodiments, it may be selected from the group consisting of amyloid-beta (Aβ) peptides, alpha synuclein, tau protein, TDP-43, human islet amyloid polypeptide (hiAPP), prion protein, and p53, and optionally from the group consisting of amyloid-beta (Aβ) peptides, alpha synuclein, tau protein, and TDP-43 . The amyloid-beta (Aβ) and alpha synuclein peptides may be monomeric, oligomeric or fibrillar peptides.

The interaction between the molecular probe and the target analyte may be detected by any suitable means detecting a biochemical interaction. The interaction may be detected by a spectroscopical method such as, but not limited to, infrared spectroscopy, UV/Vis spectroscopy, fluorescence spectroscopy, and the like.

According to one embodiment, the interaction between the molecular probe and the target analyte is detected by infrared spectroscopy, and preferably ATR-IR spectroscopy.

According to one embodiment, the interaction between the molecular probe and the target analyte is detected by infrared spectroscopy, and preferably by ATR-IR spectroscopy, and wherein the biomarker is an amide band maximum of the target analyte. The amide I band position may be an amide I band maximum of the target analyte, such as an amyloid-beta peptide or an alpha synuclein peptide, and is optionally in the range of 1600 to 1700 cm⁻¹, optionally 1620 to 1670 cm⁻¹, and optionally 1640 to 1647 cm⁻¹.

Such methods are known in the art, and are for example described in more detail below and in Nabers et al, Anal. Chem. 2016, 88, 2755-2762.

In various embodiments, said methods may involve a step of contacting the sample with an inactivated molecular probe, which is incapable of binding the target analyte, typically by mutation, but otherwise identical to the molecular probe used in the method prior to the contacting with the molecular probe immobilized on the surface of the optical element. This allows to further reduce nonspecific binding to the molecular probe. Specific examples for such inactivated molecular probes, typically antibodies with one or more mutated CDRs, with said mutations abolishing target analyte binding, as described in more detail below.

### Use of the surface-functionalized optical element

In one aspect, the present invention provides use of surface-functionalized optical element according to the invention or of an optical biosensor according to the invention for one or more of:
a companion diagnostic test,
diagnosing a protein misfolding disease, diabetes or a tumor in a patient,
monitoring of therapy of patients having a protein misfolding disease, diabetes or a tumor, and
for screening of drugs for the treatment of a protein misfolding disease, diabetes or a tumor.

The protein misfolding disease may be one or more of Alzheimer's disease, Parkinson's disease, amyotrophic lateral sclerosis, Huntington, or Prion disease.

According to one embodiment, a use of surface-functionalized optical element as defined herein or of an optical biosensor as defined herein is provided for one or more of:
diagnosing Alzheimer's disease, Parkinson's disease or amyotrophic lateral sclerosis in a patient,
monitoring of therapy of patients with Alzheimer's disease, Parkinson's disease or amyotrophic lateral sclerosis, and
screening of drugs for the treatment of Alzheimer's disease, Parkinson's disease or amyotrophic lateral sclerosis.

According to one embodiment, the surface-functionalized optical element or the optical biosensor is used for diagnosing Alzheimer's disease and/or for monitoring of therapy of patients with Alzheimer's disease.

According to one embodiment, the surface-functionalized optical element or the optical biosensor is used for diagnosing Parkinson's disease and/or for monitoring of therapy of patients with Parkinson's disease.

In one embodiment, a surface-functionalized optical element as defined herein or an optical biosensor as defined herein is provided for use in one or more of:
a companion diagnostic test,
diagnosing a protein misfolding disease, diabetes or a tumor in a patient,
monitoring of therapy of patients having a protein misfolding disease, diabetes or a tumor, and for screening of drugs for the treatment of a protein misfolding disease, diabetes or a tumor.

### Method for determining the proportion of alpha-synuclein in fibril and/or aggregated form

In another specific aspect of the methods described herein above, the present invention relates to a method for determining the proportion of alpha-synuclein in fibril and/or aggregated form relative to the total amount of alpha-synuclein in a sample obtained from a human subject, comprising:
(a) obtaining an IR spectrum of the sample in a cell comprising an infrared sensor element linked to an antibody capable of binding to alpha-synuclein independent of whether it is in its monomeric or its fibril/aggregated form, wherein said antibody binds to an epitope within amino acid residues 126-140 of human alpha-synuclein using the numbering according to SEQ ID NO:1;
(b) determining a value in the obtained infrared spectrum indicative for the proportion of alpha-synuclein in fibril and/or aggregated form relative to the total amount of alpha-synuclein in the sample.

In various preferred embodiments, the antibody used in said method is a single chain antibody (scFv) or chimeric antibody and comprises three variable heavy chain complementarity determining regions VH-CDR1, VH-CDR2 and VH-CDR3 and three variable light chain complementarity determining regions VL-CDR1, VL-CDR2 and VL-CDR3, wherein VH-CDR1 comprises the amino acid sequence of SEQ ID NO:2, VH-CDR2 comprises the amino acid sequence of SEQ ID NO:3, VH-CDR3 comprises the amino acid sequence of SEQ ID NO:4, VL-CDR1 comprises the amino acid sequence of SEQ ID NO:5, VL-CDR2 comprises the amino acid sequence of SEQ ID NO:6, and VL-CDR3 comprises the amino acid sequence of SEQ ID NO:7. "Chimeric antibody", as used herein, refers to an antibody that has been humanized in that it has a human framework region and the above CDRs.

In various embodiments, VH-CDR1, VH-CDR2, VH-CDR3, VL-CDR1, VL-CDR2 and VL-CDR3 consist of the above-given amino acid sequences, i.e. VH-CDR1 consists of the amino acid sequence of SEQ ID NO:2, VH-CDR2 consists of the amino acid sequence of SEQ ID NO:3, VH-CDR3 consists of the amino acid sequence of SEQ ID NO:4, VL-CDR1 consists of the amino acid sequence of SEQ ID NO:5, VL-CDR2 consists of the amino acid sequence of SEQ ID NO:6, and VL-CDR3 consists of the amino acid sequence of SEQ ID NO:7.

In various embodiments, the scFv comprises a heavy chain variable domain having an amino acid sequence that has at least 90% sequence identity to the amino acid sequence set forth in SEQ ID NO:8 over its entire length and a light chain variable domain having an amino acid sequences that has at least 90% sequence identity to the amino acid sequence set forth in SEQ ID NO:9 over its entire length, wherein the heavy chain variable region and the light chain variable region are covalently linked by a peptide linker of 2 to 30 amino acids in length.

In various embodiments, the amino acid sequence of the heavy chain variable domain has at least 91, 92, 93, 94, 95, 96, 97, 98, 99 or 100% sequence identity to the amino acid sequence of SEQ ID NO:8. In such embodiments, the CDR regions preferably remain invariable.

In various embodiments, the amino acid sequence of the light chain variable domain has at least 91, 92, 93, 94, 95, 96, 97, 98, 99 or 100% sequence identity to the amino acid sequence of SEQ ID NO:9. In such embodiments, the CDR regions preferably remain invariable.

In various embodiments, the scFv comprises the heavy chain variable domain (VH) and light chain variable domain (VL) as described above in any orientation, for example in VH-VL or in VL-VH orientation. In preferred embodiments, it comprises both domains in the VH-VL orientation. Such orientations of peptides are always given herein from N- to C-terminus. In various embodiments, VH and VL domains are covalently attached to each other by a peptide linker. Suitable linkers are widely used and known to those skilled in the art and typically include peptides rich in glycine and serine, generally 2 to 50 amino acids in length. In some embodiments, said linker may be 5 to 30, preferably 10 to 25 amino acids in length. It may comprise the GGS motif in one or more repeats, for example 4 to 10 copies of the GGS motif. In addition, there may be one or more other amino acids present in said linker sequence, in particular on either or both of its ends, typically only 1 or 2 amino acids other than G and S. One exemplary linker sequence is set forth in SEQ ID NO:12.

In various embodiments, the scFv comprises the structure, from N to C-terminus, V_{H}-linker-V_{L}, wherein the linker is an amino acid sequence covalently linking the V_{H} and V_{L} domain, as defined herein, which is preferably 2 to 50 amino acids in length, more preferably 5 to 30, even more preferably 10 to 25 amino acids in length, which may, in various embodiments, be rich in G and S residues, in particular the GGS motif, for example 4 to 10 copies of the GGS motif, for example having the amino acid sequence as set forth in SEQ ID NO:12.

In various embodiments, the scFv antibody comprises, from N- to C-terminus, the amino acid sequence: SEQ ID NO:8-SEQ ID NO:12-SEQ ID NO:9, covalently linked to each other by peptide bonds. In addition to said sequence, the scFv may comprise additional N- and/or C-terminal flanking sequences, typically 1 to 50 amino acids in length. These may be highly variable and may provide additional functionality, such as purification tags and or sequence stretches that facilitate expression. An exemplary N-terminal flanking sequence is set forth in SEQ ID NO:14. It is also possible to use fragments thereof. It is generally preferred that the N-terminal amino acid M is present. An exemplary C-terminal flanking sequence is set forth in SEQ ID NO:13. While said sequence includes a 6xHis tag, also fragments thereof that lack said His tag may be used. In various embodiments, said C-terminal flanking sequence comprises the GGS motif, for example 1 to 10 copies of the GGS motif, such as 2 or 3 copies. The C-terminal flanking sequence may comprise a C-terminal cysteine residue. This may allow modification or attachment to other molecules.

In various embodiments, the scFv antibody comprises or consists of the amino acid sequence set forth in SEQ ID NO:10. In various embodiments, the scFv is encoded by a nucleic acid sequence as set forth in SEQ ID NO:11.

It has further been found that nonspecific binding to the antibody may be reduced by contacting the sample with an inactivated antibody, which is incapable of binding the target analyte, typically by mutation of one or more of its CDRs, but otherwise identical to the capture antibody used in the method. The term "antibody", as used here and in the following, is intended to cover antibody fragments, such as the scFv described herein above.

Accordingly, in various embodiments, the method further comprises step (a1) of contacting the sample prior to step (a) with a variant of the antibody of step (a), wherein said variant antibody is mutated to completely abolish its binding to alpha-synuclein but wherein said antibody has at least 90%, preferably at least 95 % sequence identity relative to the antibody used in step (a) over its entire length. In various embodiments said variant of the antibody of step (a) is mutated to completely abolish its binding to alpha-synuclein but wherein said antibody has at least 91, 92, 93, 94, 95, 96, 97, 98, 99 or 100% sequence identity relative to the antibody used in step (a) over its entire length. The variant antibody may comprise any one or all, preferably all, of the mutations A3F/D in SEQ ID NO:1, R1D in SEQ ID NO:2 and G3D in SEQ ID NO:3 but is otherwise identical to the antibody used in step (a).

It is an important feature of the inventive scFv used as a capture reagent that it has high specificity for all forms of alpha-synuclein but little affinity for other proteins, for example related proteins such as beta- and gamma-synuclein. This may mean that its affinity for alpha-synuclein is significantly higher than that for any other protein, in particular beta and/or gamma-synuclein. Said difference in affinity may, in various embodiments, be at least 100-fold, but may optionally be greater such as at least 1000-fold, at least 10000-fold, at least 100000-fold or at least 1000000-fold. Said difference in affinity may help to avoid any relevant cross-reactivity with other proteins that may be present in the sample, such as beta- and/or gamma-synuclein. Accordingly, in various embodiments, under assaying conditions the affinity difference is so high and thus the cross-reactivity so low that binding to proteins other than alpha-synuclein is less than 1% of total protein bound, optionally less than 0.1%.

Further, to allow binding of the different forms of alpha-synuclein even if more than one of them are present in a sample, the antibody has the capability to bind to alpha-synuclein independent of whether it is in its monomeric or its fibril/aggregated form. This may mean that its affinity for alpha-synuclein in its monomeric, non-fibril form on the one hand and its fibril/aggregated form on the other hand is essentially the same. In various embodiments, this may mean that the affinity (dissociation constant KD) ratio of the antibody for (1) monomeric alpha-synuclein and (2) alpha-synuclein fibrils is in the range of 10:1 to 1:10, 5:1 to 1:5, 3:1 to 1:3, 2.5:1 to 1:2.5, 2:1 to 1:2, 1.8:1 to 1:1.8, 1.7:1 to 1:1.7, 1.6:1 to 1:11.6, 1.5:1 to 1:1.5, 1.4:1 to 1:1.4, 1.3:1 to 1:1.3, 1.2:1 to 1:1.2, 1.1:1 to 1:1.1 or about 1:1.

Alternatively, this missing selectivity for the different confirmations of alpha-synuclein may be measured in terms of dissociation (or "off") rates (kd). Thus, the antibodies used according to the invention may display similar dissociation rates (kd) from monomeric, non-fibril and fibril/aggregated alpha-synuclein. For example, the dissociation rate ratio may be in the range of 10:1 to 1:10, for example 5:1 to 1:5, 3:1 to 1:3, 2.5:1 to 1:2.5, 2:1 to 1:2, 1.8:1 to 1:1.8, 1.7:1 to 1:1.7, 1.6:1 to 1:11.6, 1.5:1 to 1:1.5, 1.4:1 to 1:1.4, 1.3:1 to 1:1.3, 1.2:1 to 1:1.2, 1.1:1 to 1:1.1 or about 1:1.

In certain embodiments, a binding molecule or an antibody provided herein has a dissociation constant (KD) of ≤ 1 mM, ≤ 100 nM, ≤ 10 nM, ≤ 1 nM, ≤ 0.1 nM, ≤ 0.01 nM, or ≤ 0.001 nM (e.g. 10⁻⁸ M or less, e.g. from 10⁻⁸ M to 10⁻¹³ M, e.g., from 10⁻⁹ M to 10⁻¹³ M) with respect to binding alpha-synuclein, in either (proto)fibril/aggregated or monomeric, non-fibril form.

KD and kd may be measured according to methods well known in the art, including, but not limited to, using surface plasmon resonance assays using a BIACORE^{®}-2000 or a BIACORE @-3000 (BIAcore, Inc., Piscataway, NJ) at 25°C with immobilized antigen CM5 chips at -10 response units (RU).

In various embodiments, the antibodies are characterized in that binding to either alpha-synuclein in its monomeric or aggregated/fibril form leads to a detectable difference in the amide I band as determined by IR spectroscopy, as described herein below, in particular a detectable difference in the amide I band maximum, its center of mass or the ratios of the signals at 1650 and 1630, 1656 and 1623.5 or 1651.5 and 1623.5 cm⁻¹, as described herein, in particular described herein for CSF samples.

It has been found that the requirements for the methods described herein are met by antibodies that bind epitopes in a specific region of alpha-synuclein, namely the extreme C-terminus of the native protein comprising amino acids 126-140 of SEQ ID NO:1, in particular the specific scFv antibody described herein.

It was found that such antibodies bind the different forms (conformations) of alpha-synuclein, in particular the monomeric, non-fibril form and the fibril/aggregated form, with essentially the same affinity and are less prone to interference that occurs from other proteins binding to the antibody or captured together with the alpha-synuclein (as they may be associated with alpha-synuclein in the sample). As a result, it was found that such antibodies allow the specific analysis of alpha-synuclein also before the background of complex biological samples. In contrast to this finding, other antibodies binding different epitopes were found to either not bind alpha-synuclein independent of whether it is in monomeric, non-fibril or fibril/aggregated form or to not prevent alpha-synuclein association with other proteins that may be present in the sample, which prevents the desired determination of the proportion of alpha-synuclein in fibril/aggregated form.

In various embodiments, antibodies used in the methods of the present invention are thus characterized by their property to recognize and bind an epitope within amino acid residues 126-140 of alpha-synuclein, using the numbering according to SEQ ID NO:1. The epitope may be a linear epitope. The epitope may be within the amino acids at positions 127-140, 128-135 or 131-140 of human alpha-synuclein. In some embodiments, the antibody may bind to an epitope comprising amino acids (1) 126, 127, 135, and 136, or (2) 126 and 127 or (3) 135 and 136 of human alpha-synuclein as critical residues for binding, using the positional numbering of SEQ ID NO:1. In various embodiments, the epitope bound by the antibody comprises residues 135 and 136 as critical determinants for binding. In various embodiments, the epitope may be within amino acid residues at positions 126-131, 127-132, 128-133, 129-134, 130-135, 131-136, 132-137,133-138, 134-139, 135-140, 126-130, 127-131, 128-132, 129-133, 130-134, 131-135, 132-136, 133-137, 134-138, 135-139, and 136-140. In various embodiments, the epitope consists of any one of the amino acid sequences at positions 126-131, 127-132, 128-133, 129-134, 130-135, 131-136, 132-137-,133-138, 134-139, 135-140, 126-130, 127-131, 128-132, 129-133, 130-134, 131-135, 132-136, 133-137, 134-138, 135-139, and 136-140 of human alpha-synuclein (using the positional numbering according to SEQ ID NO:1).

In various embodiments, the antibodies and variants thereof disclosed herein have a KD of 10⁻⁹ M to 10⁻¹³ M with respect to binding alpha-synuclein, in either (proto)fibril/aggregated or monomeric, non-fibril form.

In various embodiments, the antibodies and variants thereof disclosed herein have a cross-reactivity so low that binding to proteins other than alpha-synuclein is less than 1% of total protein bound, preferably less than 0.1%.

In various embodiments, the antibodies and variants thereof disclosed herein have the capability to bind to alpha-synuclein independent of whether it is in its monomeric or its fibril/aggregated form, wherein the ratio of the affinity (in terms of the dissociation constant KD) for alpha-synuclein in its monomeric, non-fibril form and for its fibril/aggregated form is in the range of 100:1 to 1:100, 80:1 to 1:80, 50:1 to 1:50, 30:1 to 1:30, 20:1 to 1:20, 10:1 to 1:10, preferably 5:1 to 1:5, more preferably 3:1 to 1:3, 2.5:1 to 1:2.5, 2:1 to 1:2, 1.8:1 to 1:1.8, 1.7:1 to 1:1.7, 1.6:1 to 1:11.6, 1.5:1 to 1:1.5, 1.4:1 to 1:1.4, 1.3:1 to 1:1.3, 1.2:1 to 1:1.2, 1.1:1 to 1:1.1 or about 1:1.

The antibodies disclosed herein may be used for determining the proportion of alpha-synuclein in fibril and/or aggregated form relative to the total amount of alpha-synuclein in a sample obtained from a human subject. Such uses of the disclosed antibodies also form part of the present invention.

In step (b) of the method, a value in the obtained infrared spectrum is determined that is indicative for the proportion of alpha-synuclein in fibril and/or aggregated form relative to the total amount of alpha-synuclein in the sample. Said value may be a band or parameter in the IR spectrum that is dependent on the presence and amount of misfolded alpha-synuclein, i.e. in fibril/aggregated form, relative to the total amount of alpha-synuclein in a sample. It is important that said value detectably differs between states in which the relative amount of misfolded alpha-synuclein is low enough to not be considered indicative for the affliction of the subject by a synucleinopathy or predictive for an increased risk to develop such and states in which it is high enough to be considered indicative or predictive for said synucleinopathy.

In various embodiments, step (b) comprises comparing the obtained IR spectrum with a reference IR spectrum of alpha-synuclein. Said reference IR spectrum may have been recorded for a sample with a known proportion of alpha-synuclein in fibril and/or aggregated form and may serve as a benchmark. Alternatively, the reference IR spectrum may have been obtained from a subject that is either known to be afflicted by or at risk of developing a synucleinopathy or has been found to be not afflicted by or at risk of developing a synucleinopathy.

It has been found that in the methods described herein, the amide I band in the IR spectrum is particularly well-suited to allow a distinction between those samples taken from individuals that are not afflicted by or at risk of developing a synucleinopathy and those samples taken from individuals that are afflicted or at risk of developing such a disease or disorder. Generally, it has been observed that the higher the proportion of misfolded alpha-synuclein in a sample, i.e. the relative amount of alpha-synuclein in fibril/aggregated form, the more the amide I band is shifted to lower values. A comparison between a reference that contain proportions of alpha-synuclein in fibril/aggregated form considered not indicative for a synucleinopathy or the risk of developing such, for example in that they originate from subjects that are not afflicted by such a disease or disorder and did not develop such in the future, and the actual sample analyzed may thus provide information that may help to diagnose or exclude an existing synucleinopathy or determine or exclude an increased risk of developing such in the future. Accordingly, in various embodiments of the methods described herein the IR spectrum recorded has sufficient signal to noise ratio to resolve at least the amide I band. The IR spectrum recorded is typically a Fourier Transform IR (FTIR) spectrum, in particular an ATR-FTIR spectrum. The method thus involves FTIR, transmission IR spectroscopy and (immuno)-ATR-FTIR spectroscopy, respectively.

The term "amide I band" as used herein, relates to a known FTIR signal observable in all peptides (including polypeptides). The amide I and amide II bands are two major bands of the protein infrared spectrum. The amide I band (typically found between 1600 and 1700 cm⁻¹) is mainly associated with the C=O stretching vibration (70-85%) and is related to the backbone conformation. In contrast, the amide II band results from the N-H bending vibration (40-60%) and from the C-N stretching vibration (18-40%).

In various embodiments, the determined value in the IR spectrum is an amide I band maximum, typically measurable at a wave number range between 1620 and 1670 cm⁻¹. In such embodiments, in particular in embodiments where a CSF sample is used, an amide I band maximum below 1639 cm⁻¹, optionally below 1638 cm⁻¹ or below 1637 cm⁻¹ or below 1636.5 cm⁻¹ may be considered indicative for the presence or an increased proportion of alpha-synuclein in fibril and/or aggregated form relative to a sample containing only the monomeric, non-fibril form of alpha-synuclein. In such embodiments, the lower the amide I band maximum, the more likely it becomes that the proportion of alpha-synuclein in fibril and/or aggregated form is pathologically relevant.

In various other embodiments, the determined value in the IR spectrum is an amide I band center of mass, again typically measurable at a wave number range between 1620 and 1650 cm⁻¹. In such embodiments, in particular in embodiments where a CSF sample is used, a center of mass of below 1641 cm⁻¹, below 1640 cm⁻¹, below 1639 cm⁻¹ or below 1638.5 cm⁻¹ may be indicative for the presence or an increased proportion of alpha-synuclein in fibril and/or aggregated form relative to a sample containing only the monomeric, non-fibril form of alpha-synuclein. In such embodiments, the lower the amide I band center of mass, the more likely it becomes that the proportion of alpha-synuclein in fibril and/or aggregated form is pathologically relevant.

In still other embodiments, the determined value in the IR spectrum is the ratio of the signal at 1650 cm⁻¹ to the signal at 1630 cm⁻¹. In such embodiments, in particular in embodiments where a CSF sample is used, a ratio of below 0.99, below 0.98, below 0.97 or below 0.96 may be indicative for the presence or an increased proportion of alpha-synuclein in fibril and/or aggregated form relative to a sample containing only the monomeric, non-fibril form of alpha-synuclein. In such embodiments, the lower the ratio, the more likely it becomes that the proportion of alpha-synuclein in fibril and/or aggregated form is pathologically relevant.

In still other embodiments, the determined value in the IR spectrum is the ratio of the signal at 1656 cm⁻¹ to the signal at 1623.5 cm⁻¹. In such embodiments, in particular in embodiments where a CSF sample is used, a ratio of below 1.15, below 1.14, below 1.10, below 1.08, below 1.07, below 1.065, below 1.06, below 1.05 or below 1.00 may be indicative for the presence or an increased proportion of alpha-synuclein in fibril and/or aggregated form relative to a sample containing only the monomeric, non-fibril form of alpha-synuclein. In such embodiments, the lower the ratio, the more likely it becomes that the proportion of alpha-synuclein in fibril and/or aggregated form is pathologically relevant.

In such preferred embodiments, where the determined value in the IR spectrum is the ratio of the signal at 1656 cm⁻¹ to the signal at 1623.5 cm⁻¹, a ratio below 1.065 is highly indicative for a disease or disorder characterized by alpha-synuclein aggregation. It has been found that a ratio below 1.065 indicates high levels of aggregated alpha synuclein, which is in turn indicative for a progressed/late stage of a disease or disorder related to alpha synuclein aggregation, such as MSA and Parkinson's disease. If the respective subject is not yet afflicted with a disease or disorder related to alpha synuclein aggregation, such a ratio is highly indicative of a high risk of developing a disease or disorder characterized by alpha-synuclein aggregation or that the onset of such disease or disorder is imminent.

In contrast to the above ratios that are indicative for a disease/disorder, a ratio of greater than 1.14 is indicative for not being afflicted by a disease or disorder characterized by alpha-synuclein aggregation or for low/reduced risk of developing a disease or disorder characterized by alpha-synuclein aggregation. Such "low" or "reduced" risk of developing a disease or disorder characterized by alpha-synuclein aggregation is understood to be relative to a subject that has a lower ratio, i.e. equal to or lower than 1.14, in particular lower than 1.065.

A ratio of the signal at 1656 cm⁻¹ to the signal at 1623.5 cm⁻¹ that is equal to or higher than 1.065 but equal to or lower than 1.14 is inconclusive in that subjects with such a ratio are typically not afflicted by a disease or disorder characterized by alpha-synuclein aggregation but have an elevated risk of developing a disease or disorder characterized by alpha-synuclein aggregation relative to those that have a ratio of greater than 1.14.

In still other embodiments, the determined value in the IR spectrum is the ratio of the signal at 1651.5 cm⁻¹ to the signal at 1623.5 cm⁻¹. In such embodiments, a ratio of below 1.20, below 1.15, below 1.10 or below 1.05 may be indicative for the presence or an increased proportion of alpha-synuclein in fibril and/or aggregated form relative to a sample containing only the monomeric, non-fibril form of alpha-synuclein. In such embodiments, the lower the ratio, the more likely it becomes that the proportion of alpha-synuclein in fibril and/or aggregated form is pathologically relevant.

In various embodiments, 2, 3, 4 or all 5 of the above three values may be determined. It has been found that the amide I band center of mass value and the ratios of the signals at 1650/1630, 1656/1623.5 and 1651.5/1623.5 cm⁻¹ are particularly suitable as the determined value, as they have been found to show the best performance, in particular in embodiments where a CSF sample is used. More preferably, the ratio of the signals at 1656/1623.5 cm⁻¹ is used.

Without wishing to be bound to any hypothesis, it is assumed that e.g. the amide I band center of mass performs particularly well as it has advantages for expectedly low analyte signals, for example relative to the absolute amide band I maximum.

The ratios are based on the principle that the determined signal at the higher fixed wave number is characteristic for alpha-synuclein with a predominantly alpha-helical and/or random coil secondary structure while the signal at the lower fixed wave number is characteristic for beta sheet secondary structures. The lower the ratio, the higher the signal characteristic for alpha-synuclein molecules with beta sheet secondary structures.

In the event more than one value is determined and there are conflicting results, the center of mass and the ratio values may thus be considered have higher reliability than the maximum value.

In the methods of the invention, the sample is contacted with the antibody in a cell that comprises the necessary instrumentation to record the IR spectrum and thus comprises at least one IR light source, an IR detection element and the actual sensor element described herein above. Said cell may be in form of a flow cell, e.g. may comprise an inlet and an outlet that allows introduction and incubation of the sample with the antibody-functionalized sensor element as well as one or more optional washing steps. These steps may be automated and may be performed continuously. In such embodiments, the cell may be part of a device that also comprises further elements, such as a pump etc.

In other embodiments, the cell is designed such that it has a single opening that is used for introduction and removal of the sample as well as, optionally, multiple washing and/or blocking solutions. In such embodiments, the introduction of the sample into the cell may also be automated but may also be done manually.

In various embodiments, introducing the sample into a cell may thus comprise bringing the sample into contact with the antibody under conditions and for a period of time that allow any alpha-synuclein present in the sample to be recognized and bound by the antibody. This typically means that the sample in liquid form is contacted with the sensor element surface on which the antibodies have been immobilized and optionally incubated for a period of time that allow binding to occur.

In various embodiments, the proportion of alpha-synuclein in fibril and/or aggregated form relative to the total amount of alpha-synuclein in the sample, or more specifically, the value indicative for said proportion may be used as a biomarker. Said biomarker may be useful for detecting and/or diagnosing a disease or disorder characterized by alpha-synuclein aggregation, for example a synucleinopathy. Additionally, such biomarker may also be useful for risk stratification in determining the risk of developing a disease or disorder characterized by alpha-synuclein aggregation. The values used may be those disclosed in connection with the inventive methods above, such as the ratio of the signal at 1656 cm⁻¹ to the signal at 1623.5 cm⁻¹ in an infrared spectrum.

The methods disclosed herein may thus be used for the diagnosis of a disease or disorder characterized by alpha-synuclein aggregation, for example a synucleinopathy, or for risk stratification, involving grading of patients into levels of risk, such as high risk and low risk or high risk, intermediate risk and low risk for developing a disease or disorder characterized by alpha-synuclein aggregation, for example a synucleinopathy. Said risk groups may be graded using the biomarkers given above, such as the signal ratios disclosed herein above. In various embodiments, the ratio of the signal at 1656 cm⁻¹ to the signal at 1623.5 cm⁻¹ is used. In some embodiments, patients with a ratio of lower than 1.065 are graded at being at high risk, patients with a ratio of higher than 1.14 graded as being a low risk and patients with a ratio between these values are graded at being at an intermediate risk. In some embodiments, patients with a ratio of lower than 1.065 are diagnosed with a disease or disorder characterized by alpha-synuclein aggregation, for example a synucleinopathy, patients with a ratio of higher than 1.14 are diagnosed as not being afflicted by a disease or disorder characterized by alpha-synuclein aggregation, for example a synucleinopathy, and patients with a ratio between these values are not clearly diagnosed as being afflicted but having an increased risk to develop such a disease or disorder.

In such use as a biomarker, the determined value indicative for the proportion of misfolded alpha-synuclein in the sample is interpreted to be indicative for a certain risk to be afflicted by or for developing a synucleinopathy. This may, for example, mean that a threshold is defined for said value, as described herein, and once said value falls below said threshold (or exceeds said threshold depending on how it is defined), the subject from which the sample has been obtained is determined to have a certain risk to be afflicted by or develop a synucleinopathy. Said determination may be considered in the diagnosis of such disease or disorder and may be combined with other diagnostic means and methods that may help to support or verify the finding.

In various embodiments, the value if used as a biomarker is thus indicative for a certain likelihood to be afflicted by or develop a synucleinopathy.

As described above, the determined value in the IR spectrum may be an amide I band maximum, an amide I band center of mass, the ratio of the signal at 1650/1630 cm⁻¹, the ratio of the signal at 1656/1623.5 cm⁻¹ and/or the ratio of the signal at 1651.5/1623.5 cm⁻¹. The threshold values described herein above can be indicative for a disease or disorder characterized by alpha-synuclein aggregation or for an increased risk of developing a disease or disorder characterized by alpha-synuclein aggregation. In all embodiments disclosed herein, said alpha-synuclein aggregation characterizing the disease or disorder may be in the brain.

In alternative embodiments, the methods of the invention may also be used to monitor or determine the efficiency of a treatment of a synucleinopathy that targets the misfolded alpha-synuclein or for screening a compound or molecule that is suspected to target misfolded alpha-synuclein. In such embodiments, the methods may be performed prior to and during or after the treatment to determine whether the proportion of alpha-synuclein in fibril and/or aggregated form relative to the total amount of alpha-synuclein in the sample has been changed by said treatment. Any change that results in a reduced proportion of alpha-synuclein in fibril and/or aggregated form may then be considered to be indicative for the success of the treatment.

Similarly, the method may be performed with samples that have been exposed to a candidate compound or molecule or with samples from an organism that has been exposed to a candidate compound or molecule, for example by administration. If said exposure of the sample or the organism from which the sample has been obtained to the candidate compound leads to a change in the proportion of alpha-synuclein in fibril and/or aggregated form relative to an untreated sample or an untreated organism, this may be considered indicative that the candidate compound of molecule is capable of interfering with the ratio of misfolded to functional alpha-synuclein, for example either by capturing and thus removing the misfolded alpha-synuclein or by preventing or decreasing such misfolding. The desirable outcome of such tests would thus be that the proportion of alpha-synuclein in fibril and/or aggregated form is decreased relative to an untreated sample or organism. The thus identified compounds and molecules may then be further developed as treatment options of the various synucleinopathies or as prophylactic treatments to prevent or delay the onset of a synucleinopathy.

"Synucleinopathy", as used herein, relates to any disease, disorder or abnormality that is associated with alpha-synuclein aggregates, fibrils, protofibrils or pathological alpha-synuclein. Known disease include, but are not limited to, Parkinson's disease (sporadic, familial with alpha-synuclein mutations, familial with mutations other than alpha-synuclein, pure autonomic failure and Lewy body dysphagia), Lewy Body dementia (LBD; dementia with Lewy bodies (DLB) ("pure" Lewy body dementia), Parkinson's disease dementia (PDD)), Diffuse Lewy Body Disease (DLBD), sporadic Alzheimer's disease, familial Alzheimer's disease with APP mutations, familial Alzheimer's disease with PS-1, PS-2 or other mutations, familial British dementia, Lewy body variant of Alzheimer's disease, multiple system atrophy (MSA; Shy-Drager syndrome, striatonigral degeneration and olivopontocerebellar atrophy), inclusion-body myositis, traumatic brain injury, chronic traumatic encephalopathy, dementia pugilistica, tauopathies (Pick's disease, frontotemporal dementia, progressive supranuclear palsy, corticobasal degeneration, Frontotemporal dementia with Parkinsonism linked to chromosome 17 and Niemann-Pick type C1 disease), Down syndrome, Creutzfeldt-Jakob disease, Huntington's disease, motor neuron disease, amyotrophic lateral sclerosis (sporadic, familial and ALS-dementia complex of Guam), neuroaxonal dystrophy, neurodegeneration with brain iron accumulation type 1 (Hallervorden-Spatz syndrome), prion diseases, Gerstmann-Straussler-Scheinker disease, ataxia telangiectatica, Meige's syndrome, subacute sclerosing panencephalitis, Gaucher disease, Krabbe disease as well as other lysosomal storage disorders (including Kufor-Rakeb syndrome and Sanfilippo syndrome), or rapid eye movement (REM) sleep behavior disorder. More particularly, the synucleinopathy may be selected from Parkinson's Disease, Multiple System Atrophy (MSA), Lewy Body dementia (LBD; dementia with Lewy bodies (DLB) ("pure" Lewy body dementia), Parkinson's disease dementia (PDD)), and Diffuse Lewy Body Disease, optionally from Lewy body disease, MSA and Parkinson's Disease, for example from MSA and PD.

The alpha-synuclein aggregates may include Lewy bodies, Lewy neurites and/or glial cytoplasmic inclusions.

In various embodiments, the sample used in the methods described herein is a biological sample. Such biological samples include body fluid samples, such as, in particular, a cerebrospinal fluid sample, a blood sample, a serum sample, or a plasma sample. The sample may also be a tissue sample, for example in form of a homogenate, such as a brain tissues sample or skin tissue sample. The samples may be complex biological samples, such as those described above, that contain multiple other components and which the alpha-synuclein may only be contained in minute amounts. The samples may be used without any pretreatment. In other words, the samples, for example blood samples, may be used as obtained, i.e. unprocessed, without the need to isolate or concentrate the analyte. This allows use of the methods in clinical applications. Blood samples include full blood samples, as well as blood plasma or serum samples.

### Optical Biosensor

In one aspect, the invention provides an optical biosensor which comprises the surface-functionalized optical element as defined herein.

In various embodiments, the sensor element may comprise an internal reflection element, in particular an attenuated total internal reflection element. Said internal reflection element may have a trapezoid or parallelogram shape, hemi-cylindrical, polyhedral, fiber or rod-shaped geometry, for example a trapezoid shape. Said internal reflection element may be a (mono)crystal of any suitable material that is sufficiently transparent to IR light (i.e. light with a wavelength of between 780 nm and 1 mm or at least between 780 nm and 25 µm). In various embodiments, the material used includes, but is not limited to plastic, glass, diamond, gold, silver, palladium, germanium, silicon, silicon dioxide, silver halides, GaAs, ZnSe, ZnS, thallium(I) mixed halides (e.g. KRS-5), and AMTIRs. "AMTIR" is an amorphous material transmitting infrared radiation which is known in the art. A suitable AMTIR may be Ge33As12Se55 (AMTIR-1). Particularly suitable materials include germanium, gallium arsenide, silicon, zinc selenide, and diamond. These materials may be used in form of crystals, for example monocrystals. In various embodiments, the material is therefore a silicon crystal or a germanium crystal, for example a silicon crystal.

In various embodiments, the internal reflection element is arranged such that it allows for more than one passage of IR light through the reflection element. The number of passages in such embodiments may range from 2 to 20 or more, optionally 3 to 30 or 4 to 25, such as at least 5 passages. The higher number of passages ensures that a sufficient signal-to-noise ratio is obtained. In various embodiments, 20-30 reflections may be used, optionally with 10 to 20 actively sensed reflections. This may for example be achieved by using a multireflection crystal internal reflection element, for example in trapezoid or parallelogram shape.

The surface-functionalized optical biosensor element comprises a capture reagent immobilized on its surface that is able to bind the target analyte in the sample and thus immobilize it on the sensor element surface. In the methods described herein, the capture reagent is a molecular probe as defined herein above which is covalently attached to the surface of the optical element as described herein above.

The (molecular probe can interact with the target analyte to create a physical-chemical change, which is transformed into a measurable signal by the transduction element. The signal is measured by optical spectroscopy such as, but not limited to, infrared ATR spectroscopy, transmission spectroscopy, Raman spectroscopy, colorimetric microscopy, fluorescent microscopy, luminescent microscopy, or combinations of these techniques.

Hence, the optical biosensor according to the invention can be used in conjunction with infrared ATR spectroscopy, transmission spectroscopy, Raman spectroscopy, colorimetric microscopy, fluorescent microscopy, luminescent microscopy, or combinations of these techniques.

According to one preferred embodiment, the device is a transmission IR-based biosensor or an ATR-IR-based biosensor, and more preferably the device is an ATR-IR-based biosensor. Such devices are known in the field, and are for example described in Nabers et al, Anal. Chem. 2016, 88, 2755-2762.

In various methods of the invention, alpha-synuclein is analyzed by IR spectroscopy to record an IR spectrum which is then analyzed to determine a value indicative for the proportion of alpha-synuclein in fibril and/or aggregated form relative to the total amount of alpha-synuclein in the sample. To record such an IR spectrum, the sample including the alpha-synuclein is introduced into a cell that may comprise an IR source, an IR detector and an IR-based biosensor element. The general setup of an IR spectrometer is generally known. In the present methods, the IR-based biosensor element is that part of the setup that is specifically designed to allow determining the proportion of the different conformational forms of alpha-synuclein in the sample. The sensor element may be a surface-functionalized optical biosensor element described herein above.

In one particularly preferred embodiment the invention is thus directed to a surface-functionalized optical biosensor element, in particular an infrared sensor element, such as for determining the proportion of alpha-synuclein in fibril and/or aggregated form relative to the total amount of alpha-synuclein in a sample obtained from a human subject, with said sensor element comprising an antibody as defined herein. All embodiments of the antibodies and sensor elements used in the methods disclosed herein similarly apply to the sensor elements of the invention. The antibody is covalently attached to the sensor.

In one aspect said optical biosensor element of the invention may comprise an internal reflection element, for example having a trapezoid or parallelogram shape. Said internal reflection element may be a (mono)crystal of any suitable material that is transparent to IR light and the suitable materials include, but are not limited to germanium, gallium arsenide, silicon, zinc selenide, and diamond. As described in relation to the inventive methods above, the antibody is generally immobilized such that its antigen-binding regions are accessible to the sample and come into contact with any potential analyte that may be comprised therein. In various embodiments, the antibody is immobilized to one surface of the sensor element, more specifically the internal reflection element, with said surface lying opposite to the surface facing the IR source and thus the incident IR light. This is the typical setup for ATR-FTIR spectroscopy and leads to a situation where the incident light enters the internal reflection element and interacts with the antibody (covalently) bound to the opposite surface before being reflected and passing through the internal reflection element again, with said passage, interaction and reflection being repeated multiple times to improve the S/N ratio.

The sensor elements of the invention may be used for determining the proportion of alpha-synuclein in fibril and/or aggregated form relative to the total amount of alpha-synuclein in a sample obtained from a human subject. Such use as well as the use in the methods described herein forms one different aspect of the present invention.

Further, the sensor elements may also be used for detecting a disease or disorder characterized by alpha-synuclein aggregation or for determining the risk of developing a disease or disorder characterized by alpha-synuclein aggregation. This may include determining the proportion of alpha-synuclein in fibril and/or aggregated form relative to the total amount of alpha-synuclein in a sample obtained from a human subject, as described herein above.

While the optical biosensor has been described herein above with reference to use for the detection of alpha synuclein in a sample, it is understood that it may similarly be used for other target analytes, such as those mentioned herein above. It is also understood that for such different target analytes a suitable molecular probe needs to be selected.

All embodiments described herein for any one of the methods, sensor elements, devices and uses of the invention, are similarly applicable for the respective other aspects of the invention and vice versa. All documents cited herein are incorporated by reference in their entirety. The invention is further illustrated by the following, non-limiting examples.

### EXAMPLES

### Example 1: Preparation of the charge optimized sensor element using a charge optimization moiety.

### A: Preparation of the charge optimized sensor element according to one embodiment of the invention

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Surface attachment moiety: | 1-azido-N-(3-(triethoxysilyl)propyl)-3,6,9,12,15-pentaoxaoctadecan-18-amide |
| Spacer moiety: | 3-(2-(2-methoxyethoxy)ethoxy)-N-(3-(triethoxysilyl)propyl)propanamide |
| Charge optimization moiety: | 3-amino-1-(2-azatricyclo[10.4.0.0^{4,9}]hexadeca-1(16),4,6,8,12,14-hexaen-10-yn-2-yl)propan-1-one |
| Linker moiety: | 2,5-dioxopyrrolidin-1-yl 1-{2-azatricyclo[10.4.0.04,9]hexadeca-4,6,8,12,14,16-hexaen-10-yn-2-yl}-1,4-dioxo-7,10,13,16,19,22,25,28,31,34,37,40,43-tridecaoxa-3-aza-hexatetracontan-46-oate |
| Antibody labeling moiety: | 2,5-dioxopyrrolidin-1-yl 1-azido-3,6,9,12,15,18,21,24-octaoxaheptacosan-27-oate |
| Antibody 1: | anti-β-amyloid (13-28) antibody, IgG, mouse monoclonal |
| Antibody 2: | anti-β-amyloid (dead mutant) antibody, IgG, mouse monoclonal |
| Device: | ATR-IR-biosensor |

### Pretreatment of the silicon crystal

The surface was activated by an oxidative treatment (plasma treatment) to produce a stable silicon dioxide layer.

### Silanization of a silicon crystal

For silanization of the silicon crystal, a mixture of the surface attachment moiety and the spacer moiety in 2-propanol was prepared and contacted with a selected surface of the silicon crystal. The crystal was subsequently rinsed with H₂O and dried under nitrogen.

### Production of DBCO-labeled antibody

The anti-β-amyloid antibody (molecular probe 1 or 2) was reacted with the antibody labeling moiety for preparing a azide-labeled anti-β-amyloid antibody as the molecular probe.

### Functionalization of surface with the charge optimization moiety and immobilization of antibody

The crystal surface previously silanized with a mixture of the surface attachment moiety and the spacer moiety, was further reacted with the charge optimization moiety in a SPAAC click reaction to obtain an amine-functionalized surface.

The coupling takes place by reaction of the DBCO group of the charge optimization moiety with the azide group on the silanized surface. After SPAAC click reaction, amine groups are available on the silanized silicon crystal.

For this, the silanized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with an acetonitrile/water mixture (25:75 v/v). Subsequently, the charge optimization moiety was solved in the acetonitrile/water mixture and circulated over the crystal surface, followed by a washing step with the acetonitrile/water mixture in flow-through mode. During the amine-functionalization process, complete IR spectra were continuously recorded and referenced against the acetonitrile/water background.

After silanization and amine-functionalization by the SPAAC click reaction, the surface was further reacted with the linker moiety in flow-through mode to obtain DBCO-functionalization. The reaction was performed in PBS and complete IR spectra were continuously recorded and referenced against the PBS background. Next, excess NHS groups of the linker moiety were quenched with an ethanolamine solution followed by another washing step.

In a next step, the previously prepared azide-labeled anti-β-Amyloid antibody (molecular probe 1 or 2) was immobilized on the DBCO-functionalized and charge-optimized surface by a SPAAC click reaction between the DBCO moiety on the surface and the azide group of the labeled antibody in flow-through mode. During antibody attachment, complete IR spectra were continuously recorded and compared with the background before antibody attachment. An indication for successful immobilization of the antibody on the surface is the amide 2 signal (1550 cm⁻¹).

The functionalized surface prepared in this process corresponds to a charge optimized sensor element according to one embodiment of the invention, also referred to herein as "charge optimized amine surface" or just "amine surface".

### B. Preparation of comparative sensor element without charge optimization

A comparative sensor element was prepared implementing a peptide blocking layer. Compared to the previously described charge optimized sensor element, the comparative sensor element replaces the charge optimization moiety with a peptide linker moiety. After reaction with the peptide linker moiety, the comparative sensor element obtained reactive NHS groups which can be further reacted with peptide compound 1 and 2. Said sensor elements are essentially in accordance with the disclosure in WO2024003214A2 and WO2024003213A1.

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Surface attachment moiety: | 1-azido-N-(3-(triethoxysilyl)propyl)-3,6,9,12,15-pentaoxaoctadecan-18-amide |
| Spacer moiety: | 3-(2-(2-methoxyethoxy)ethoxy)-N-(3-(triethoxysilyl)propyl)propanamide |
| Peptide linker moiety: | 2,5-dioxopyrrolidin-1-yl 6-{2-azatricyclo[10.4.0.0^{4,9}]hexadeca-4,6,8,12,14,16-hexaen-10-yn-2-yl}-6-oxohexanoate |
| Peptide compound 1: | casein fragment blocking solution, made from bovine casein (Sigma Aldrich / Art.-Nr.: C3400) by alkaline hydrolysis |
| Peptide compound 2: | BSA-free peptide blocking solution, made from chemically modified peptides (Candor Bioscience / Art.-Nr.: 113) |
| Linker moiety: | 2,5-dioxopyrrolidin-1-yl 1-{2-azatricyclo[10.4.0.0^{4,9}]hexadeca-4,6,8,12,14,16-hexaen-10-yn-2-yl}-1,4-dioxo-7,10,13,16,19,22,25,28,31,34,37,40,43-tridecaoxa-3-aza-hexatetracontan-46-oate |
| Antibody labeling moiety: | 2,5-dioxopyrrolidin-1-yl 1-azido-3,6,9,12,15,18,21,24-octaoxaheptacosan-27-oate |
| Antibody 1: | anti-β-amyloid (13-28) antibody, IgG, mouse monoclonal |
| Antibody 2: | anti-β-amyloid (dead mutant) antibody, IgG, mouse monoclonal |
| Device: | ATR-IR-biosensor |

### Pretreatment and silanization of the silicon crystal

Pretreatment and silanization with the surface attachment moiety and the spacer moiety were carried out as described above for inventive example 1 A.

### Blocking of surface and immobilization of antibody on peptide blocking layer

The peptide linker moiety was reacted in a SPAAC click reaction with the previously silanized crystal surface. The coupling takes place by reaction of the DBCO group with the azide group on the silanized surface. After SPAAC click reaction, reactive NHS groups are available on the silanized silicon crystal which can be reacted with peptide compounds 1 and/or 2.

After silanization and NHS-activation by the SPAAC click reaction, a protein-reactive silicon crystal was available. In the subsequent surface functionalization, the NHS-functionalized crystal surface was first blocked with either peptide compound 1 or 2. Covalent bonding can be achieved via amide bonding of the primary amine group of the lysines of the peptide compound or the N-termini of the peptide compound with the NHS-functional groups of the silicon crystal.

The NHS-functionalized crystal was placed with the top (long side) facing up on the crystal holder of the ATR flow-through cuvette. The lid of the cuvette with inserted silicone seal was placed on the crystal. The cuvette was placed on the cuvette holder in the IR spectrometer. The sample compartment of the spectrometer was sealed and purged with dry air. The flow system including the cuvette was flushed with water and then with PBS buffer. Subsequently the casein fragment blocking solution was circulated over the crystal surface, followed by a washing step with PBS in flow-through mode. During the blocking process, complete IR spectra were continuously recorded and referenced against the PBS background. The blocking process was monitored by following an increase of the amide 2 absorbance (1550 cm⁻¹) and simultaneously a decrease of NHS absorbance (1740 cm⁻¹) in time. Next, excess NHS groups were quenched with an ethanolamine solution followed by another washing step.

Subsequently, the peptide blocking layer was DBCO-functionalized by reaction with the NHS- and DBCO-containing linker moiety in flow-through mode.

Immobilization of the anti-β-amyloid antibody (molecular probe 1 or 2) was carried out as described above for inventive example 1 A. This element will also be described as "casein-based blocking surface" with peptide compound 1 or as "BSA-free peptide blocking surface" with peptide compound 2.

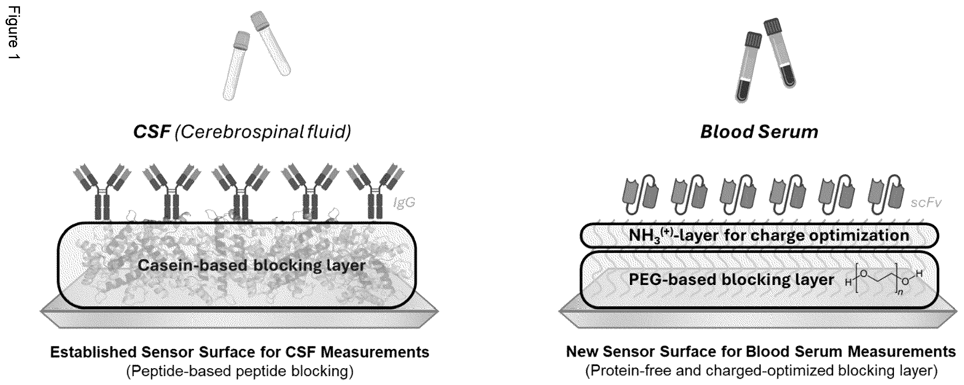

A schematic overview of the described surfaces in Example 1A and 1B is shown in **Figure 1****.**

### C. Inertness test and determination of surface charge

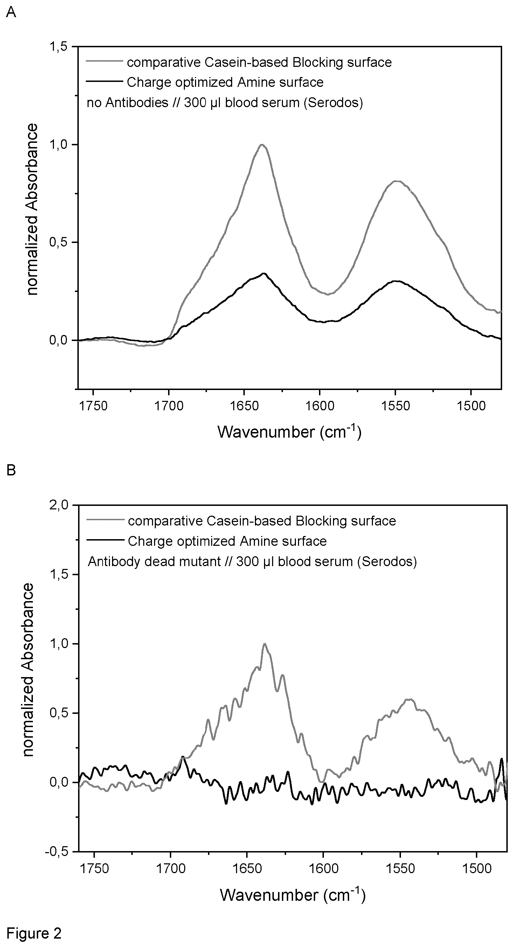

**Figure 2A** shows the result after the blood serum was circulated for 60 minutes over the functional surfaces prepared in examples 1A and 1B without a molecular probe attached. Both surfaces were then washed for another 120 minutes with PBS buffer solution in flow-through mode. In said Figure, the functional surface according to the invention is referred to as "Amine-Surface", the comparative surface with peptide compound 1 is referred to as "casein-based blocking surface".

During both steps, IR spectra were continuously recorded, revealing that overall the NSP (nonspecific protein) adsorption is strongly reduced for the surface of the inventive example compared to the comparative example.

**Figure 2B** reveals the effect of a molecular probe (molecular probe 2) coupled to the functionalized surface with respect to NSP adsorption. The results show that NSP adsorption is overall reduced by the antibody presence for both surfaces, resulting in non-detectable NSP adsorption on the "Amine Surface" and reduced nonspecific protein binding for the comparative surface.

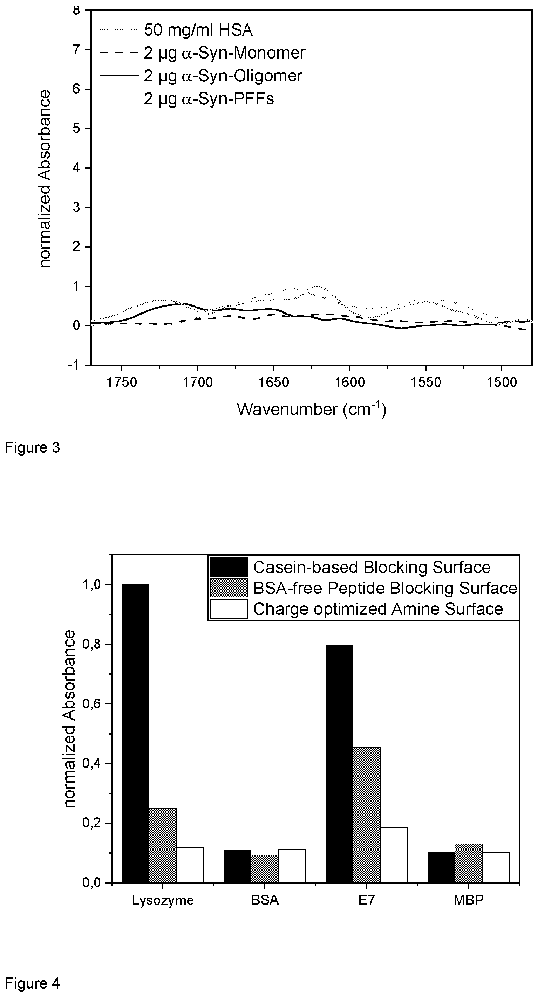

In another experimental setup, the surface of the inventive example was exposed to human serum albumin (HSA), α-Synuclein-monomer, α-Synuclein-oligomer and α-Synuclein-fibrils (PFFs) to investigate the NSP adsorption onto the surface if no antibody is present. **Figure 3** shows that essentially no NSP adsorption occurs.

In another experiment the surface inertness was tested with especial focus on the charge influence of the surfaces. In order to compare the surface of the invention two comparative surfaces were generated where instead of the charge optimization moiety, conventional peptide-based blocking moieties were used (Example 1B). All sensor surfaces were functionalized with antibody 1/molecular probe 1. The absorption of the amide II band was monitored and the results are shown in **Figure 4****.** Four different proteins were used to investigate the inertness of the respective surfaces of which two have a positive net charge (Lysozyme, E7) and two have a negative net charge (bovine serum albumin (BSA), maltose binding protein (MBP)) under the buffer conditions used (PBS pH 7.4). **Figure 4** shows that there are significant differences in adsorption of proteins which have a positive charge, underlining the optimized charge properties of the Amine Surface.

### Example 2: Characterization of a scFv-5757 functionalized charge-optimized optical element

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Surface attachment moiety: | 1-azido-N-(3-(triethoxysilyl)propyl)-3,6,9,12,15-pentaoxaoctadecan-18-amide |
| Spacer moiety: | 3-(2-(2-methoxyethoxy)ethoxy)-N-(3-(triethoxysilyl)propyl)propanamide |
| Charge optimization moiety: | 3-amino-1-(2-azatricyclo[10.4.0.0^{4,9}]hexadeca-1(16),4,6,8,12,14-hexaen-10-yn-2-yl)propan-1-one |
| Linker moiety: | 2,5-dioxopyrrolidin-1-yl 1-{2-azatricyclo[10.4.0.0^{4,9}]hexadeca-4,6,8,12,14,16-hexaen-10-yn-2-yl}-1,4-dioxo-7,10,13,16,19,22,25,28,31,34,37,40,43-tridecaoxa-3-aza-hexatetracontan-46-oate |
| Antibody labeling moiety: | 2,5-dioxopyrrolidin-1-yl 1-azido-3,6,9,12,15,18,21,24-octaoxaheptacosan-27-oate |
| Antibodies: | scFv with the amino acid sequence set forth in SEQ ID NO:10 (5757scFv), Mouse Anti-alpha-synuclein IgG with the same variable domains as the scFv (VH and VL domains as set forth in SEQ ID Nos. 8 and 9) either glycosylated (5757nativ) or deglycosylated (5757degly), |
| Device: | ATR-IR-biosensor |

The surface was activated and functionalized as described in Example 1A and 1B, the only difference being the use of a different antibody for the detection of α-Synuclein.

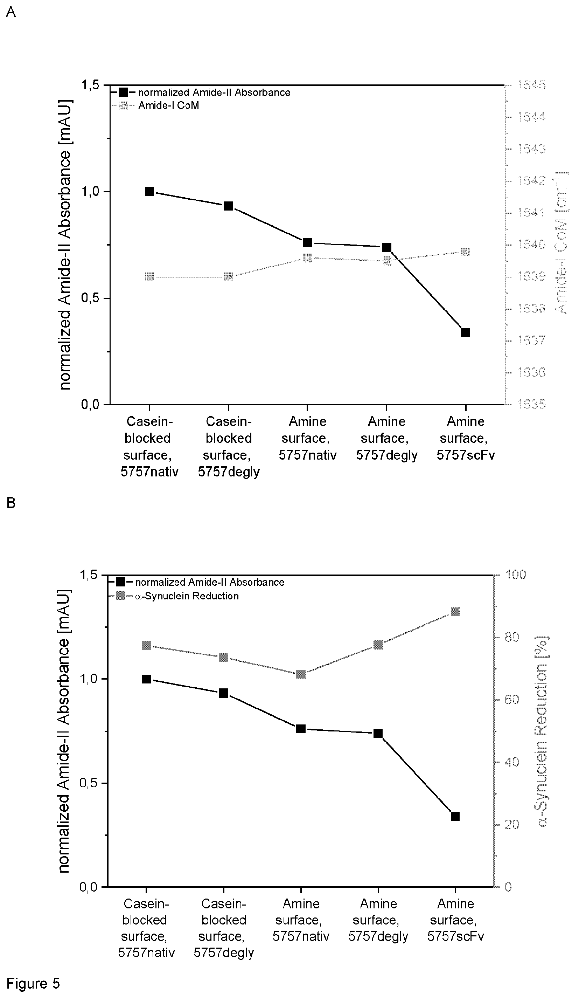

Characterization was performed by analyzing plasma samples on the surfaces described in Example 1A and 1B, modified with different antibodies, to show the improved characteristics of the new developed surface (Example 1A). Plasma samples were circulated on the surfaces in a flow through system for 60 minutes and then washed with PBS buffer for another 60 minutes. The results are shown in Figure 5A and B, where the focus lays on reducing the nonspecific binding to the surface and the improved target binding. Resulting values were taken from the infrared spectra of the wash step (Figure 5A) and from the analyzed supernatant (Figure 5B, α-Syn Reduction). The supernatant was analyzed via Enzyme-linked Immunosorbent Assay (ELISA, BioLegend^{®} LEGEND MAX^{™} Human α-Synuclein Colorimetric ELISA KIT, Art-Nr. 448607)

**Figure 5A** **and** **B** show the effect of different surface functionalization on NSP adsorption from a plasma sample. All surfaces which are modified versions of Example 1A (referred to as "Amine Surface") exhibit reduced amide-II absorbance due to reduced nonspecific binding. Interestingly, the scFv of the invention show significantly less NSP adsorption.

**Figure 5A** also shows that the amide-I signal (target signal) is not significantly changed by the different surface functionalizations. Small changes indicate the higher portion of the specific target binding compared to the reference surface (referred to as "Casein-based Blocking Surface").

**Figure 5B** further shows that the surface functionalization according to the invention (referred to as "Amine Surface, 5757scFv") captures almost 90% of the target analyte (a-Synuclein). Compared to comparative with alternative surface functionalizations and different antibodies, the surface of the invention exhibit a strongly increased capability to capture the target analyte from the sample solution while showing the smallest value in Amide-II absorbance. This underlines the improvement in specific target binding.

### Example 3: Binding characteristics of scFv-5757 and respective mutants

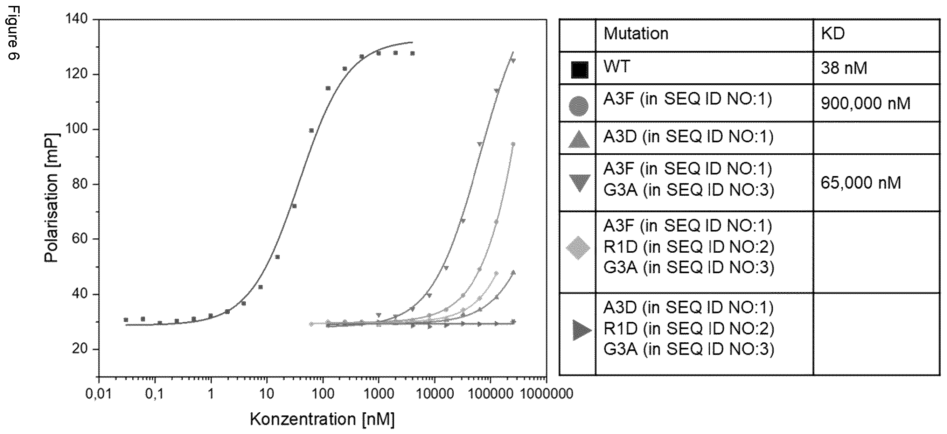

*Ab initio* structure prediction of an antibody comprising VH (SEQ ID NO: 8) and VL (SEQ ID NO: 9) binding to α-synuclein monomer using AlphaFold 2, revealed that three amino acid residues of the heavy chain CDRs are particularly involved in the binding to α-Synuclein: A3 in SEQ ID NO: 1, R1 in SEQ ID NO: 2 and G3 in SEQ ID NO: 3. **Figure 6** shows that single and combinatorial mutations of these residues significantly decrease the binding affinity of the antibody. For several mutations the determination of a K_{D} was not possible (A3D in SEQ ID NO: 1, A3F in SEQ ID NO: 1, R1D in SEQ ID NO: 2 and G3D in SEQ ID NO: 3, A3D in SEQ ID NO: 1, R1D in SEQ ID NO: 2 and G3D in SEQ ID NO: 3). These mutants were thus chosen for non-binding variants of the antibody used as the capture reagent, e.g. to be used in sample treatment to eliminate nonspecific binding to the antibody used as a capture reagent before the sample is contacted with the capture antibody.

### Example 4: Improved antigen binding capabilities of functionalized charge-optimized optical elements

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Surface attachment moiety: | 1-azido-N-(3-(triethoxysilyl)propyl)-3,6,9,12,15-pentaoxaoctadecan-18-amide |
| Spacer moiety: | 3-(2-(2-methoxyethoxy)ethoxy)-N-(3-(triethoxysilyl)propyl)propanamide |
| Charge optimization moiety: | 3-amino-1-(2-azatricyclo[10.4.0.0^{4,9}]hexadeca-1(16),4,6,8,12,14-hexaen-10-yn-2-yl)propan-1-one |
| Linker moiety: | 2,5-dioxopyrrolidin-1-yl 1-{2-azatricyclo[10.4.0.0^{4,9}]hexadeca-4,6,8,12,14,16-hexaen-10-yn-2-yl}-1,4-dioxo-7,10,13,16,19,22,25,28,31,34,37,40,43-tridecaoxa-3-aza-hexatetracontan-46-oate |
| Antibody labeling moiety: | 2,5-dioxopyrrolidin-1-yl 1-azido-3,6,9,12,15,18,21,24-octaoxaheptacosan-27-oate |
| Antibody: | anti-β-amyloid (1-5) antibody, IgG, mouse monoclonal |
| Device: | ATR-IR-biosensor |

The surface was activated and functionalized as described in Example 1A and 1B, differentiating in use of a different antibody for the detection of β-amyloid-(1-42)-globulomer.

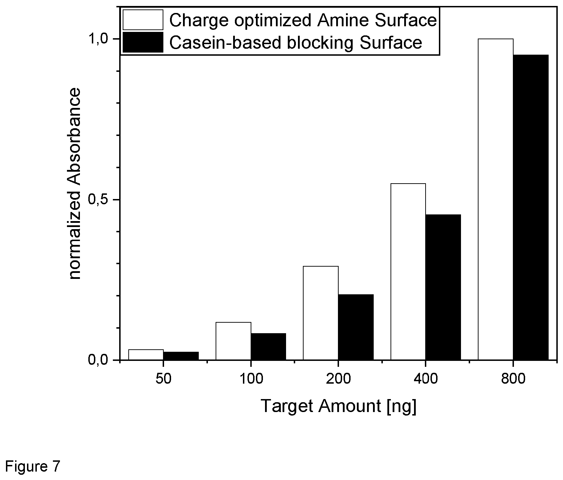

The functional surface prepared (1A) was exposed to the target analyte (β-amyloid-(1-42)-globulomer) to investigate target analyte binding to the surface compared to a comparative surface, prepared according to Example 1B using peptide compound 1 and subsequently also coupled to the same antibody. The target analyte was circulated in PBS buffer with rising target concentrations in a flow through setup. The results in **Figure 7** indicate the amount of surface bound target analyte represented by the amide-II band of the target analyte.

**Figure 7** shows the result after the target analyte was circulated over the functional surfaces (1A and 1B) with stepwise addition of the target analyte. Each step took 60 minutes of circulation over the surfaces. Result values were taken from the last part of each circulation step where amide-II values of the infrared signals were saturated. The functional inventive surface is referred to as "Amine-Surface", the comparative surface as "Casein-based blocking Surface". The "Amine-Surface" exhibited increased target analyte binding for all analyte concentrations, which was particularly pronounced at lower analyte concentrations.

### Example 5: Analysis of clinical samples for alpha-synuclein using surfaces and antibodies of the invention

### Materials

| | |
|---|---|
| Optical element: | Silicon ATR Crystal (internal reflection element), trapezoidal, incidence angle 45°, 52mm x 20mm x 2mm (Tol: +/- 0.1mm), optically polished |
| Surface attachment moiety: | 1-azido-N-(3-(triethoxysilyl)propyl)-3,6,9,12,15-pentaoxaoctadecan-18-amide |
| Spacer moiety: | 3-(2-(2-methoxyethoxy)ethoxy)-N-(3-(triethoxysilyl)propyl)propanamide |
| Charge optimization moiety: | 3-amino-1-(2-azatricyclo[10.4.0.0^{4,9}]hexadeca-1(16),4,6,8,12,14-hexaen-10-yn-2-yl)propan-1-one |
| Linker moiety: | 2,5-dioxopyrrolidin-1-yl 1-{2-azatricyclo[10.4.0.0^{4,9}]hexadeca-4,6,8,12,14,16-hexaen-10-yn-2-yl}-1,4-dioxo-7,10,13,16,19,22,25,28,31,34,37,40,43-tridecaoxa-3-aza-hexatetracontan-46-oate |
| Antibody labeling moiety: | 2,5-dioxopyrrolidin-1-yl 1-azido-3,6,9,12,15,18,21,24-octaoxaheptacosan-27-oate |
| Antibody: | Anti-alpha-synuclein scFv with the amino acid sequence set forth in SEQ ID NO:10 |
| Device: | ATR-IR-biosensor |

The surface was activated and functionalized as described in Example 2.

The functionalized surface prepared was exposed to individual human blood serum samples to investigate the diagnostic performance of the biosensor. Human blood serum samples were circulated over the surface in a flow through setup and then washed with PBS buffer solution. Each sample was applied in a 1:1 mixture with the sample additive AssayDefender^{®} (CANDOR^{®} Bioscience, Art.-Nr. 180050) in a total volume of 500 µl. The sample circulation step took 60 minutes each and sample wash was performed in a duration of 120 minutes. As a result, the mean value of the amide-I absorption band was taken from the first 60 minutes of the wash step to distinguish between the different serum samples.

The serum samples were divided into two different groups where a diseased group consisted of samples from patients with Morbus Parkinson (PD) or multi system atrophy (MSA), both known as typical synucleinopathies. A group of control serum samples without any known synucleinopathy was taken as a healthy control group. Hereby, the diseased group consisted of 14 individual serum samples and the control group of 23 individual serum samples.

It could be shown that the two different groups can clearly be distinguished with an area under curve (AUC) of 0.87 (data not shown). This underlines the diagnostic performance potential of the inventive surface and the inventive antibody (Example 1A) with individual human blood serum samples.

## Claims

1. A surface-functionalized optical element comprising
an IR-transparent optical element,
wherein at least a part of the surface of the IR-transparent optical element is surface-functionalized, wherein said surface functionalization comprises
a surface attachment moiety covalently attached to the surface of the IR-transparent optical element, and
wherein at least part of the surface attachment moieties is covalently attached to a spacer moiety comprising a poly(alkylene oxide),
wherein at least part of the spacer moieties is covalently attached to a charge optimization moiety, wherein said charge optimization moiety comprises a functional group that is charged under conditions of use of the optical element and optionally a poly(alkylene oxide),
wherein at least part of the charge optimization moiety is covalently attached to a linker moiety comprising a poly(alkylene oxide),
wherein at least part of the linker moieties is covalently attached to a molecular probe.

2. The surface-functionalized optical element according to claim 1, wherein said charge optimization moiety comprises a functional group that is at least charged, preferably positively charged, if said charge optimization moiety is not covalently attached to a linker moiety, optionally wherein at least part but not all of the charge optimization moieties are covalently attached to a linker moiety.

3. The surface-functionalized optical element according to claim 2, wherein the number of the charge optimization moieties not covalently attached to a linker moiety is adjusted such that the net charge of the surface of the surface-functionalized optical element is about zero.

4. The surface-functionalized optical element according to any one of the preceding claims, wherein said surface functionalization comprises
(1) a charge optimization construct having the structure
//-(S)-(SP)-(CO)
wherein
// represents attachment to the surface of the IR-transparent optical element,
(S) represents the surface attachment moiety,
(SP) represents the spacer moiety,
(CO) represents the charge optimization moiety,
wherein said charge optimization moiety comprises a functional group that is charged under conditions of use of the optical element; and/or
(2) a molecular probe linker construct having the structure
//-(S)-(SP)-(CO)-(L)-\\
wherein
// represents attachment to the surface of the IR-transparent optical element,
(S) represents the surface attachment moiety,
(SP) represents the spacer moiety,
(CO) represents the charge optimization moiety,
(L) represent the linker moiety, and
\\ represents attachment to another moiety of the molecular probe linker construct or to the molecular probe.

5. The surface-functionalized optical element according to any one of the preceding claims, wherein
(1) the surface attachment moiety (S) has the structure
//-Y¹-L¹-X¹-$$¹
wherein
// represents the attachment to the surface of the IR-transparent optical element,
Y¹ is -O-Si(R¹)₂-, wherein R¹ is each independently selected from H, OH, optionally substituted alkyl, optionally substituted alkoxy, -O-//, and -O-Si^{x}, wherein Si^{x} is a silicon atom of another silicon-containing surface attachment moiety or a silicon atom of an optional matrix silane being attached to the surface of the IR-transparent optical element,
L¹ is an optional linking unit,
X¹ is a group selected from -C(O)NH-, -NHC(O)-, -NHC(O)HN-, and -NHC(O)O-,
$$¹ represents attachment to the spacer moiety; and/or
(2) the spacer moiety (SP) has the structure
$$²-L²-X²-L³-H¹-&&²
wherein
$$² represents attachment to the surface attachment moiety (S),
L² and L³ are each independently an optional linking unit,
X² is -(AO)n¹-, wherein AO is an alkylene oxide, preferably ethylene oxide (EO) or propylene oxide (PO), more preferably ethylene oxide, and n¹ is an integer from 2 to 25, preferably from 2 to 10, more preferably from 2 to 8, most preferably from 3 to 5,
H¹ is an optionally substituted N-heterocyclic group having a structure according to any one of formulae (HET-I) to (HET-VI): 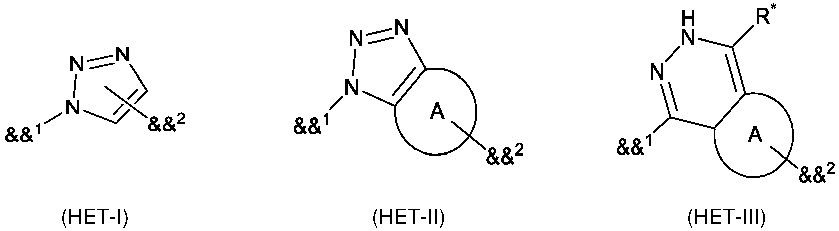 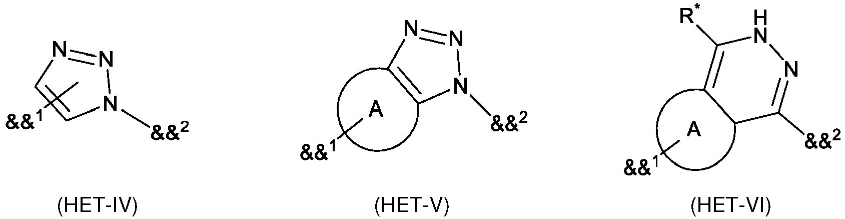 wherein
&&¹ represents attachment to L³ or, if L³ is absent, to X²,
&&² represents the attachment to the charge optimization moiety (CO),
R* is H, an optionally substituted alkyl group or an optionally substituted aryl group, and preferably is a C1-C6 alkyl group,
ring A is an optionally substituted 8- to 9-membered mono-, bi- or multicyclic carbocycle or an optionally substituted 8- to 9-membered mono-, bi- or multicyclic heterocycle, and is preferably an optionally substituted dibenzo-fused 8-membered N-heterocycle or an optionally substituted bicyclo[6.1.0]non-4-ene ring, more preferably an optionally substituted bicyclo[6.1.0]non-4-ene ring; and/or
(3) the linker moiety (L) has the structure
##²-L⁷-X⁷-L¹⁰-H²-L¹¹-**
or
(##²-L⁷-X⁷-L⁸)₂X⁸-L⁹-X⁹-L¹⁰- H²-L¹¹-**
wherein
each ##² represents attachment to the charge optimization moiety (CO),
L⁷, L⁸, L⁹, L¹⁰ and L¹¹ are each independently an optional linking unit,
X⁷ is -(AO)n³-, wherein AO is an alkylene oxide, preferably ethylene oxide (EO) or propylene oxide (PO), more preferably ethylene oxide, and n³ is an integer from 1 to 25, preferably from 2 to 10, more preferably from 3 to 9,
X⁸ is NC(O)-, NC(O)NH- or NC(O)O-,
X⁹ is -(AO)n⁴-, wherein AO is an alkylene oxide, preferably ethylene oxide (EO) or propylene oxide (PO), more preferably ethylene oxide, and n⁴ is an integer from 1 to 25, preferably from 2 to 10, more preferably from 2 to 5, most preferably 2 to 4,
wherein the sum of all n³ and n⁴ is preferably 4 to 25, more preferably 5 to 12,
H² is an optionally substituted N-heterocyclic group having a structure according to any one of formulae (HET-I) to (HET-VI): 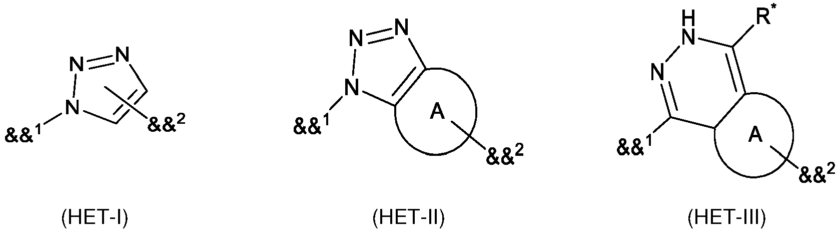 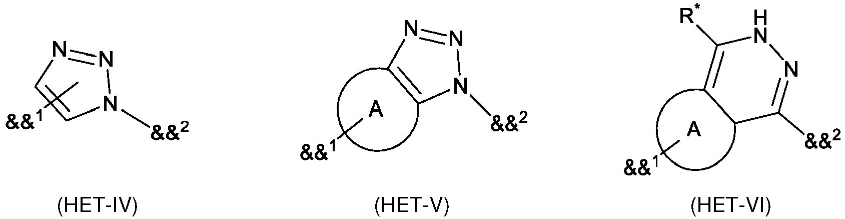 wherein
&&¹ represents attachment to L¹⁰ or, if L¹⁰ is absent, to X⁹,
&&² represents the attachment to L¹¹ or, if L¹¹ is absent, corresponds to **,
R* is H, an optionally substituted alkyl group or an optionally substituted aryl group, and preferably is a C₁-C₆ alkyl group,
ring A is an optionally substituted 8- to 9-membered mono-, bi- or multicyclic carbocycle or an optionally substituted 8- to 9-membered mono-, bi- or multicyclic heterocycle, and is preferably an optionally substituted dibenzo-fused 8-membered N-heterocycle or an optionally substituted bicyclo[6.1.0]non-4-ene ring, more preferably an optionally substituted dibenzo-fused 8-membered N-heterocycle, and
** represents the attachment to another moiety of the molecular probe linker construct or to the molecular probe,
wherein, optionally, the sum of all n¹, n², n³, and n⁴ of the molecular probe linker construct is in the range of 10 to 25, preferably 12 to 18.

6. The surface-functionalized optical element according to any one of the preceding claims, wherein the charge optimization moiety (CO) has the structure
&&³-L⁴-X³-L⁵-X⁴-L⁶-C¹
wherein
&&³ represents attachment to the spacer moiety (SP),
L⁴, L⁵ and L⁶ are each independently an optional linking unit,
X³ is a group selected from -C(O)NH-, -NHC(O)-, -NHC(O)HN-, -NHC(O)O-, and -OC(O)NH-,
X⁴ is absent or, preferably, is -(AO)n²-, wherein AO is an alkylene oxide, preferably ethylene oxide (EO) or propylene oxide (PO), more preferably ethylene oxide, and n² is an integer from 1 to 25, preferably from 2 to 10, more preferably from 2 to 5, most preferably 2 to 4,
C' is (1) a charged functional group, preferably an amine group, more preferably a primary amine group, or (2) has the structure
X⁶-##¹
wherein X⁶ is covalently attached to a L⁶ and is a group selected from -C(O)NH-, -NHC(O)-, -NHC(O)HN-, -NHC(O)O-, and -OC(O)NH-, and
##¹ represents attachment to the linker moiety (L),
wherein, optionally, the charge optimization moiety (CO) has the structure
-(CH₂)₁₋₂-OC(O)-NH-(CH₂CH₂O)₂₋₄-(CH₂)₂₋₄-NH₃⁺
or
-(CH₂)₁₋₂-OC(O)-NH-(CH₂CH₂O)₂₋₄-(CH₂)₂₋₄-NHC(O)-##¹.

7. The surface-functionalized optical element according to any one of the preceding claims, wherein the molecular probe is an antigen, an antibody, a fragment of an antibody, an antibody fusion protein or fusion proteins with antibody fragments, an antibody conjugate or conjugates with antibody fragments, a protein complex which optionally contains an antibody fragment or a fusion protein thereof, an anticalin, a nanobody, a small organic molecule, a drug, a nucleic acid, an aptamer, a lipid, a carbohydrate, or a peptide, optionally a single chain variable fragment (scFv) comprising, from N- to C-terminus, the amino acid sequence: SEQ ID NO:8-SEQ ID NO:12-SEQ ID NO:9, covalently linked to each other by peptide bonds, said single chain variable fragment preferably comprising or consisting of the amino acid sequence of SEQ ID NO:10.

8. Single chain variable fragment (scFv) comprising, from N- to C-terminus, the amino acid sequence: SEQ ID NO:8-SEQ ID NO:12-SEQ ID NO:9, covalently linked to each other by peptide bonds, said scFv preferably comprising or consisting of the amino acid sequence of SEQ ID NO:10.

9. An optical biosensor or infrared sensor element comprising the surface-functionalized optical element according to any one of claim 1 to 7 and/or the single chain variable fragment of claim 8.

10. Method for detecting a biomarker, comprising the steps of
(i) bringing into contact a surface-functionalized optical element according to any one of claims 1 to 7 or the optical biosensor or infrared sensor element of claim 9 with a sample suspected to comprise a target analyte, and optionally wherein the sample is a human body fluid;
(ii) detecting the biomarker based on an interaction between the molecular probe and the target analyte, and optionally wherein the interaction between the molecular probe and the target analyte is detected by infrared spectroscopy, and optionally wherein the biomarker is based on an amide I band position of the target analyte.

11. Use of a surface-functionalized optical element according to any one of claims 1 to 7 or of an optical biosensor or infrared sensor element according to claim 9 for one or more of:
a companion diagnostic test,
diagnosing a protein misfolding disease, diabetes or a tumor in a patient,
monitoring of therapy of patients having a protein misfolding disease, diabetes or a tumor, and
for screening of drugs for the treatment of a protein misfolding disease, diabetes or a tumor.

12. A method for determining the proportion of alpha-synuclein in fibril and/or aggregated form relative to the total amount of alpha-synuclein in a sample obtained from a human subject, comprising:
(d) obtaining an IR spectrum of the sample in a cell comprising an infrared sensor element linked to an antibody capable of binding to alpha-synuclein independent of whether it is in its monomeric or its fibril/aggregated form, wherein the antibody is a single chain antibody (scFv) and comprises three variable heavy chain complementarity determining regions VH-CDR1, VH-CDR2 and VH-CDR3 and three variable light chain complementarity determining regions VL-CDR1, VL-CDR2 and VL-CDR3, wherein VH-CDR1 comprises the amino acid sequence of SEQ ID NO:2, VH-CDR2 comprises the amino acid sequence of SEQ ID NO:3, VH-CDR3 comprises the amino acid sequence of SEQ ID NO:4, VL-CDR1 comprises the amino acid sequence of SEQ ID NO:5, VL-CDR2 comprises the amino acid sequence of SEQ ID NO:6, and VL-CDR3 comprises the amino acid sequence of SEQ ID NO:7, wherein optionally the scFv antibody comprises a heavy chain variable domain (V_{H}) having an amino acid sequence that has at least 90%, preferably 100%, sequence identity to the amino acid sequence set forth in SEQ ID NO:8 over its entire length and a light chain variable domain (V_{L}) having an amino acid sequences that has at least 90%, preferably 100%, sequence identity to the amino acid sequence set forth in SEQ ID NO:9 over its entire length, wherein the heavy chain variable region and the light chain variable region are covalently linked by a peptide linker of 2 to 50 amino acids in length, wherein preferably the scFv is according to claim 10;
(e) determining a value in the obtained infrared spectrum indicative for the proportion of alpha-synuclein in fibril and/or aggregated form relative to the total amount of alpha-synuclein in the sample.

13. The method of claim 12, wherein the method further comprises step (a1) of contacting the sample prior to step (a) with a variant of the antibody of step (a), wherein said variant antibody is mutated to completely abolish its binding to alpha-synuclein but wherein said antibody has at least 90%, preferably at least 95 % sequence identity relative to the antibody used in step (a) over its entire length, wherein, optionally, the variant antibody comprises the mutations A3F/D in SEQ ID NO:1, R1D in SEQ ID NO:2 and G3D in SEQ ID NO:3 but is otherwise identical to the antibody used in step (a).

14. The method of claim 12 or 13, wherein
(1) the value indicative for the proportion of alpha-synuclein in fibril and/or aggregated form determined in step (b) is
(b1) an amide I band maximum;
(b2) an amide I band center of mass;
(b3) a ratio of signal at 1650 cm⁻¹ to signal at 1630 cm⁻¹;
(b4) a ratio of signal at 1656 cm⁻¹ to signal at 1623.5 cm⁻¹; or
(b5) a ratio of signal at 1651.5 cm⁻¹ to signal at 1623.5 cm⁻¹;
and/or
(2) step (b) comprises comparing the obtained IR spectrum with a reference IR spectrum of alpha-synuclein, wherein optionally the reference IR spectrum is obtained from a sample with a known proportion of alpha-synuclein in fibril and/or aggregated form;
and/or
(3) the method further comprises based on the value indicative for the proportion of alpha-synuclein in fibril and/or aggregated form relative to the total amount of alpha-synuclein in the sample, determining whether the human subject is afflicted by or has a risk of developing a disease or disorder **characterized by** alpha-synuclein aggregation,
wherein, optionally, (c1) an amide I band maximum below 1639 cm⁻¹, optionally below 1638 cm⁻¹, (c2) an amide I band center of mass of below 1641 cm⁻¹, optionally below 1640 cm⁻¹, (c3) a ratio of the signal at 1650 cm⁻¹ to the signal at 1630 cm⁻¹ below 0.99, optionally below 0.98, (c4) a ratio of the signal at 1656 cm⁻¹ to the signal at 1623.5 cm⁻¹ below 1.15, optionally below 1.10, and/or (c5) a ratio of the signal at 1651.5 cm⁻¹ to the signal at 1623.5 cm⁻¹ below 1.20, optionally below 1.15,
is indicative for a disease or disorder **characterized by** alpha-synuclein aggregation or for an increased risk of developing a disease or disorder **characterized by** alpha-synuclein aggregation;
and/or
(4) the sample is a sample obtained from a human subject suspected of being afflicted by a disease or disorder **characterized by** alpha-synuclein aggregation or at risk of developing such a disease or disorder, wherein the disease or disorder is optionally selected from Lewy body disease and Parkinson's disease; and/or
(5) the sample is a cerebrospinal fluid sample, a blood sample, or a tissue homogenate including brain and skin.

15. Use of
(1) a scFv antibody comprising three variable heavy chain complementarity determining regions VH-CDR1, VH-CDR2 and VH-CDR3 and three variable light chain complementarity determining regions VL-CDR1, VL-CDR2 and VL-CDR3, wherein VH-CDR1 comprises the amino acid sequence of SEQ ID NO:2, VH-CDR2 comprises the amino acid sequence of SEQ ID NO:3, VH-CDR3 comprises the amino acid sequence of SEQ ID NO:4, VL-CDR1 comprises the amino acid sequence of SEQ ID NO:5, VL-CDR2 comprises the amino acid sequence of SEQ ID NO:6, and VL-CDR3 comprises the amino acid sequence of SEQ ID NO:7, wherein optionally the scFv antibody comprises a heavy chain variable domain (V_{H}) having an amino acid sequence that has at least 90%, preferably 100%, sequence identity to the amino acid sequence set forth in SEQ ID NO:8 over its entire length and a light chain variable domain (V_{L}) having an amino acid sequences that has at least 90%, preferably 100%, sequence identity to the amino acid sequence set forth in SEQ ID NO:9 over its entire length, wherein the heavy chain variable region and the light chain variable region are covalently linked by a peptide linker of 2 to 50 amino acids in length, wherein preferably the scFv is according to claim 10, for determining the proportion of alpha-synuclein in fibril and/or aggregated form relative to the total amount of alpha-synuclein in a sample obtained from a human subject; or
(2) of the optical biosensor or infrared sensor element of claim 9 for
(a) determining the proportion of alpha-synuclein in fibril and/or aggregated form relative to the total amount of alpha-synuclein in a sample obtained from a human subject; or
(b) for detecting a disease or disorder **characterized by** alpha-synuclein aggregation or for determining the risk of developing a disease or disorder **characterized by** alpha-synuclein aggregation by determining the proportion of alpha-synuclein in fibril and/or aggregated form relative to the total amount of alpha-synuclein in a sample obtained from a human subject.
